# EUROPEAN PATENT SPECIFICATION

(11) **EP 2 430 176 B1**
(45) Date of publication and mention of the grant of the patent: **07.08.2024**
(21) Application number: 10720052.9
(22) Date of filing: 08.04.2010
(51) Int. Cl.: C12P 19/04, C12P 19/14, C12N 9/28, C12N 9/44

(54) **IMPROVED PRODUCTION OF MALTOTETRAOSE SYRUP USING A PSEUDOMONAS SACCHAROPHILA MALTOTETRAOHYDROLASE VARIANT AND A DEBRANCHING ENZYME**
VERBESSERTE MALTOTETRAOSESIRUP PRODUKTION UNTER VERWENDUNG EINER PSEUDOMONAS SACCHAROPHILA MALTOTETRAOHYDROLASE VARIANTE UND EINES "DEBRANCHING" ENZYMS
PRODUCTION AMÉLIORÉE DE SIROP DE MALTOTÉTRAOSE À L'AIDE D'UN VARIANT DE MALTOTÉTRAOHYDROLASE DE PSEUDOMONAS SACCHAROPHILA ET D'UNE ENZYME DÉBRANCHANTE

(30) Priority: 11.05.2009 US 177136 P
(43) Date of publication of application: 21.03.2012
(73) Proprietor: Danisco US Inc., Palo Alto, CA 94304 (US)
(72) Inventor: DUAN, Gang, Palo Alto, CA 94304 (US); QIAN, Ying, Palo Alto, CA 94304 (US); SALA, Rafael, F., Palo Alto, CA 94304 (US); SHETTY, Jayarama, Palo Alto, CA 94304 (US)
(74) Representative: Mewburn Ellis LLP
(86) International application number: PCT/US2010/030443
(87) International publication number: WO 2010/132157

(56) References cited:
- EP-A1- 0 605 040
- WO-A2-2006/003461
- WO-A2-2007/148224
- WO-A2-2010/118269
- US-A- 6 074 854

## Description

### FIELD OF THE INVENTION

A variant maltotetraohydrolase from *Pseudomonas saccharophila* and a pullulanase are useful for production of a saccharide syrup from a starch liquefact, among other things.

### BACKGROUND

The conversion of vegetable starches, especially cornstarch, to maltotetraose and lower sugars, such as glucose or maltose, is a rapidly expanding industry. The current process consists of two sequential enzyme-catalyzed steps that result in the production of glucose or maltose. The first enzyme-catalyzed step is starch liquefaction. Typically, a starch suspension is gelatinized by rapid heating to 85°C or more. Alpha-amylases (EC 3.2.1.1) are used to degrade the viscous liquefact to maltodextrins. Alpha-amylases are endohydrolases that catalyze the random cleavage of internal α-1, 4-D-glucosidic bonds. As alpha-amylases break down the starch, the viscosity decreases. Because liquefaction typically is conducted at high temperatures, thermostable alpha-amylases, such as an alpha-amylase from *Bacillus* sp., are preferred for this step.

A second enzyme-catalyzed saccharification step is required to break down the maltodextrins. Glucoamylases and/or maltogenic alpha-amylases commonly are used to catalyze the hydrolysis of non-reducing ends of the maltodextrins formed after liquefaction, releasing D-glucose, maltose and isomaltose.

Maltotetraose (G4 or DP4) syrup is one of many commercially important products derived from enzymatic treatment of starch. G4 syrup has a number of advantageous properties compared to sucrose syrups. For example, partially replacing sucrose with G4 syrup in a food reduces the food's sweetness without affecting its taste or flavor. G4 syrup has high moisture retention in foods and exhibits less deleterious Maillard reaction products because of its lower glucose and maltose content. G4 syrup also has higher viscosity than sucrose, thus improving food texture. G4 syrup depresses the freezing point of water less than sucrose or high fructose syrup, so G4 syrup can better control the freezing points of frozen foods. After ingestion, G4 syrup also affects osmotic pressure less than sucrose. Together, these qualities make G4 syrup ideally suited as an ingredient in foods and medical products. G4 syrup is useful in other industries, as well. For example, G4 syrup imparts gloss and can be used advantageously as a paper sizer. *See, e.g*., Kimura et al., "Maltotetraose, a new saccharide of tertiary property," Starch 42: 151-57 (1990).

*Pseudomonas saccharophila* expresses a useful G4-forming amylase variously known as *P. saccharophila* maltotetraohydrolase, "Amy3A," "PSA," "SAS," or "PS4." SAS and PS4 are used interchangeably herein. PS4 displays both endo- and exo-alpha-amylase activities. While endo-alpha-amylase activity is useful for decreasing the viscosity of gelatinized starch, exo-alpha-amylase activity is particularly useful for breaking down maltodextrins to smaller saccharides, such as G4. SAS has been subject to extensive genetic engineering to create various SAS variants with improved properties useful for G4 syrup production. A particular variant, SAS3, has shown promising features for G4 syrup production. *See*, *e.g*., WO 07/148224 (Danisco A/S). Nevertheless, the starch material is not efficiently utilized in SAS3-catalyzed G4 syrup productions, as a significant amount of DP5+ (oligosaccharides with a degree of polymerization of 5 or above) is present in the final product. Accordingly, there is still need to improve the G4 syrup production, e.g., by reducing the amount of DP5+, to maximize the utilization of the starch substrate.

WO 2006/003461 discloses a PS4 variant polypeptide derivable from a parent polypeptide having non-maltogenic exoamylase activity in which the PS4 variant polypeptide comprises an amino acid mutation at one or more positions selected from the group consisting of: consisting of: 121, 161, 223, 146, 157, 158, 198, 229, 303, 306, 309, 316, 353, 26, 70, 145, 188, 272, 339, with reference to the
position numbering of a *Pseudomonas saccharophilia* exoamylase sequence shown as SEQ ID NO: 1.

EP 0605040A1 discloses a heat-stable pullulanase, which has the property of hydrolysing alpha-1,6 glycosidic bonds in amylopectin and which has enzymatic activity in acidic medium and at a temperature of approximately 60°C.

### SUMMARY

A *Pseudomonas saccharophila* maltotetraohydrolase (PS4) variant is capable of producing maltotetraose from either liquefied starch or other source of maltodextrins at a high temperature, *e.g*., about 60°C to about 75°C. A significant amount of higher oligosaccharides, however, remains at the end of the PS4-catalyzed saccharification. To improve the efficiency of maltotetraose production, a pullulanase is supplemented to the PS4 variant containing composition during saccharification. Such a combination is able to produce more maltotetraose, *e.g*., about 20% more than using the PS4 variant alone.

The present invention provides a method of processing a starch comprising saccharifying a starch liquefact or a maltodextrin by contacting a *Pseudomonas saccharophila* amylase (PS4) variant and a pullulanase to the starch liquefact or the maltodextrin to form a saccharide syrup, wherein the PS4 variant has at least 80% amino acid sequence identity to SEQ ID NO:2 and has amino acid deletions, additions, insertions, or substitutions compared to the amino acid sequence of SEQ ID NO: 2, or comprises up to 25 amino acid deletions, additions, insertions, or substitutions compared to the amino acid sequence of SEQ ID NO: 2, and wherein the pullulanase comprises an amino acid sequence having at least about 80% amino acid sequence identity to SEQ ID NO: 6, wherein the saccharide syrup comprises at least about 54% by weight maltotetraose based on total saccharide content.

In some embodiments, the PS4 variant comprises a G223E amino acid substitution compared to the PS4 having an amino acid sequence of SEQ ID NO: 2. In some embodiments the PS4 variant comprises an amino acid sequence having up to 15 additional amino acid substitutions compared to the PS4 having an amino acid sequence of SEQ ID NO: 2. In some embodiments the PS4 variant comprises one or more of the following amino acid substitutions: N33Y, D34N, G70D, G121F, G134R, A141P, Y146G, I157L, S161A, L178F, A179T, S229P, H307K, A309P, and/or S334P compared to the PS4 having an amino acid sequence of SEQ ID NO: 2. In some embodiments the PS4 variant comprises an amino acid sequence of SEQ ID NO: 3.

In some embodiments, the PS4 variant exhibits altered properties compared to the PS4 having an amino acid sequence of SEQ ID NO: 2. In some embodiments the altered properties include improved thermostability and/or improved stability at a pH of 5.0 to 7.0 compared to the PS4 having an amino acid sequence of SEQ ID NO: 2. In some embodiments the altered properties include an increased exo-alpha-amylase activity or a decreased endo-alpha-amylase activity compared to the PS4 having an amino acid sequence of SEQ ID NO: 2.

In some embodiments, the pullulanase is a naturally occurring enzyme from *Bacillus*, and optionally from *Bacillus deramificans.* In some embodiments, the pullulanase comprises an amino acid sequence of SEQ ID NO: 6; or the pullulanase consists of an amino acid sequence of SEQ ID NO: 6; or the pullulanase is a variant enzyme, optionally a variant of a *Bacillus deramificans* pullulanase having an amino acid sequence of SEQ ID NO: 6 and having amino acid deletions, additions, insertions, or substitutions compared to the amino acid sequence of SEQ ID NO: 6. In some embodiments the variant pullulanase exhibits altered properties compared to a pullulanase having an amino acid sequence of SEQ ID NO: 6. In some embodiments, the altered properties include improved thermostability, pH dependent activity, specific activity, substrate specificity, or any combination thereof, compared to a pullulanase having an amino acid sequence of SEQ ID NO: 6.

In some embodiments, the pullulanase is expressed and/or isolated from a host cell of *Bacillus*, and optionally from the host cell of *Bacillus licheniformis.* In some embodiments, a Carlsberg protease gene and/or a Glu C protease gene of the host cell has been altered to eliminate protease activity.

In some embodiments, the PS4 variant and/or the pullulanase are purified.

In some embodiments, the PS4 variant is added to the starch liquefact in a range from 0.05 to 1 Kg/Metric Tons Dry Solids.

In some embodiments, the pullulanase, measured as Kg/Metric Tons Dry Solids, is added to the starch liquefact at an amount in a range from 5 to about 50 times of the PS4 variant.

In some embodiments, (a) the starch liquefact is saccharified at 60°C to 75°C; and/or (b) the starch liquefact is saccharified at pH 3.9 to pH 5.5.

In some embodiments, the saccharide syrup contains at least 20% more maltotetraose than a saccharide syrup obtained with the PS4 variant but without the pullulanase.

In some embodiments, the method further comprises contacting an isoamylase, a protease, a cellulase, a hemicellulase, a lipase, a cutinase, or any combination thereof, to the starch liquefact or the maltodextrin.

In some embodiments, the starch is from corn, wheat, barley, rye, milo, sago, cassava, tapioca, sorghum, rice, peas, bean, banana, or potatoes.

### BRIEF DESCRIPTION OF THE DRAWINGS

The accompanying drawings are incorporated into the specification and provide non-limiting illustrations of various embodiments. In the drawings:

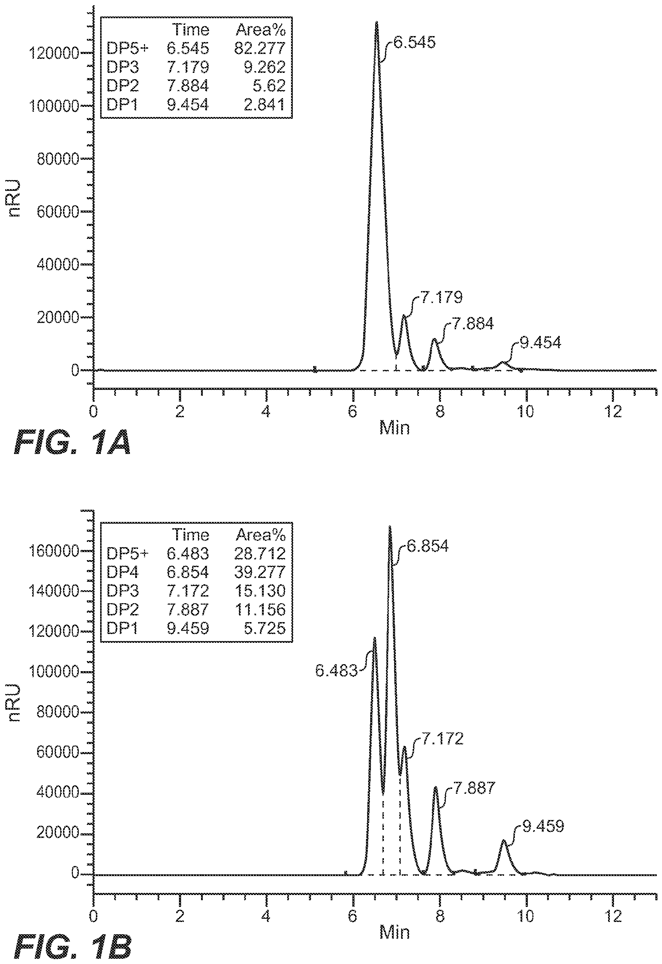

FIG. 1A depicts the chromatogram showing the oligosaccharide profile of the liquefact supernatant before SAS3 was added. No detectable amount of DP4 is present in the liquefact. FIG. 1B depicts the chromatogram showing the oligosaccharide profile of the liquefact supernatant 24.2 hours after the addition of 0.048 Kg/MTDS SAS3. The retention time and the relative amount of each oligosaccharide were shown on the top right corner. The relative amount of each oligosaccharide was calculated based on the area under the corresponding peak. DP5+ collectively appeared as a single peak under the present detection condition.

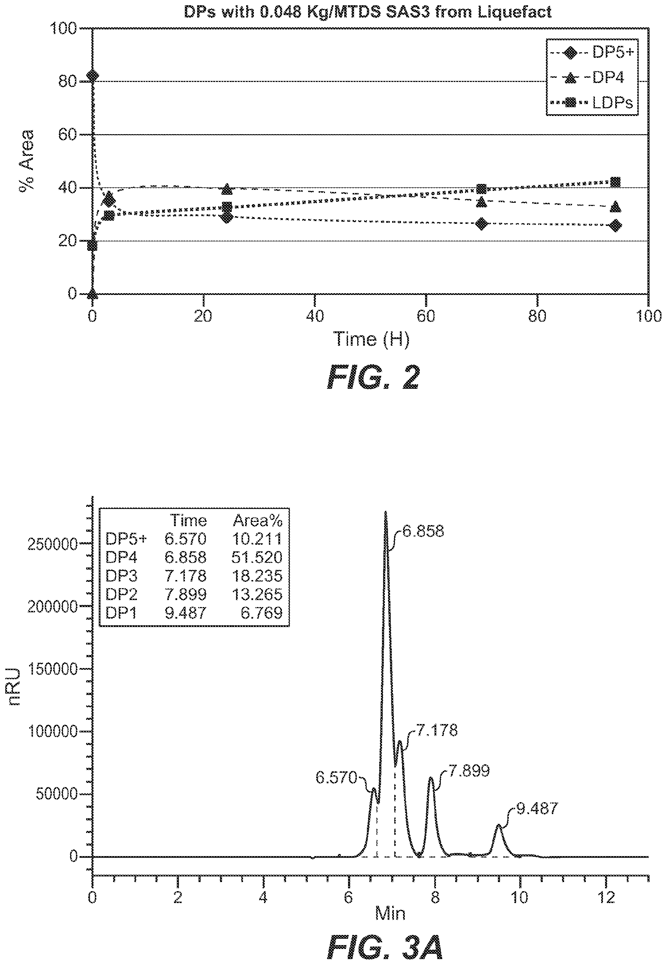

FIG. 2 depicts the oligosaccharide profile of the liquefact supernatant at various time points after adding 0.048 Kg/MTDS SAS3. LDPs collectively reflected DP1, DP2, and DP3.

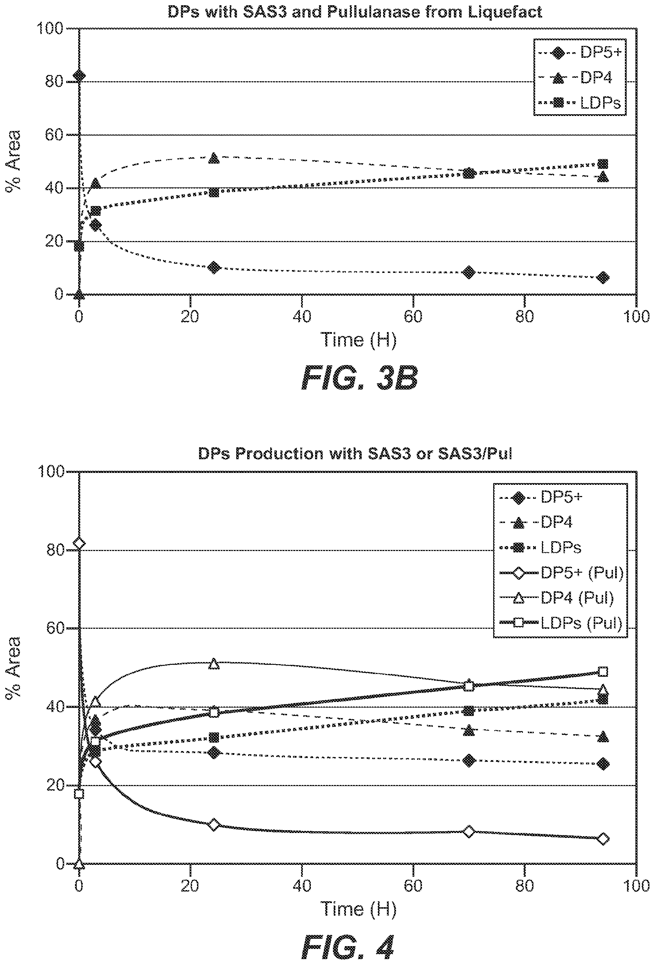

FIG. 3A depicts the chromatogram showing the oligosaccharide profile of the liquefact supernatant 24.2 hours after the addition of 0.046 Kg/MTDS SAS3 and 0.24 Kg/MTDS pullulanase. There is a significant increase of DP4 compared to FIG. 1B. FIG. 3B depicts the oligosaccharide profile of the liquefact supernatant at various time points after adding 0.046 Kg/MTDS SAS3 and 0.24 Kg/MTDS pullulanase.
FIG. 4 depicts the oligosaccharide profile of the liquefact supernatant at various time points after adding 0.048 Kg/MTDS SAS3 or 0.046 Kg/MTDS SAS3 plus 0.24 Kg/MTDS pullulanase.

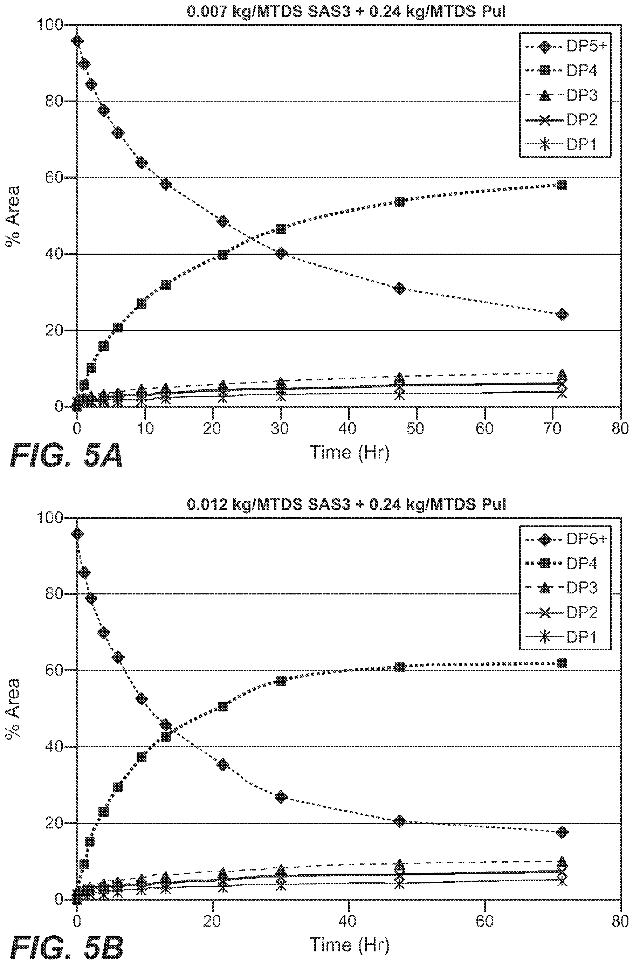

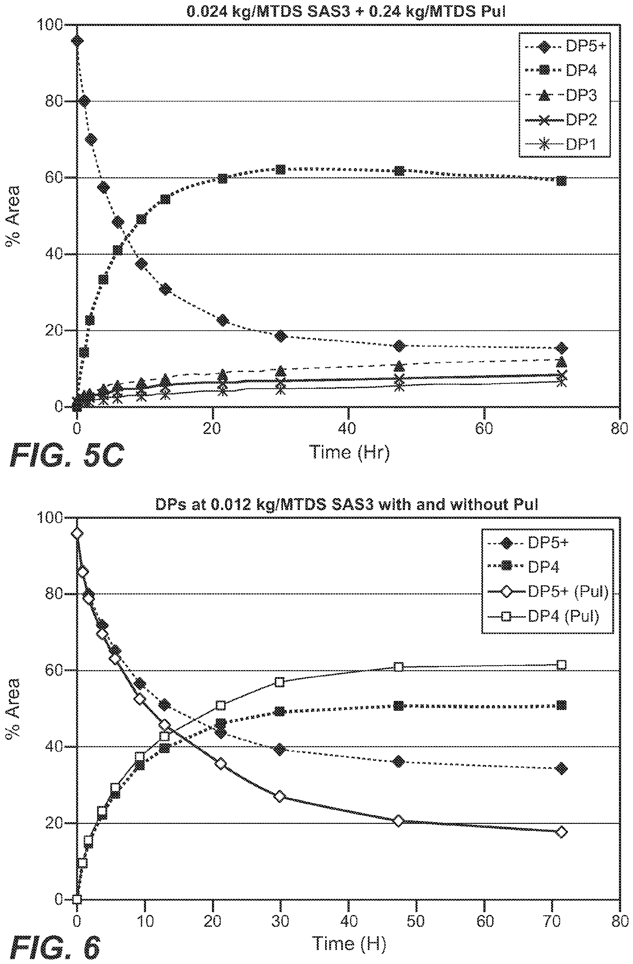

FIG. 5A depicts the oligosaccharide profile at various time points after Maltrin^{®} M040 is treated with 0.007 Kg/MTDS SAS3 and 0.24 Kg/MTDS pullulanase. FIG. 5B depicts the oligosaccharide profile at various time points after Maltrin^{®} M040 is treated with 0.012 Kg/MTDS SAS3 and 0.24 Kg/MTDS pullulanase. FIG. 5C depicts the oligosaccharide profile at various time points after Maltrin^{®} M040 is treated with 0.024 Kg/MTDS SAS3 and 0.24 Kg/MTDS pullulanase.
FIG. 6 depicts the comparison of DP5+ hydrolysis and DP4 production catalyzed by SAS3 with and without supplementation of the pullulanase. The amounts of DP5+ and DP4 are plot against the time. Maltodextrin (Maltrin^{®} M040) is used herein as the saccharification substrate.

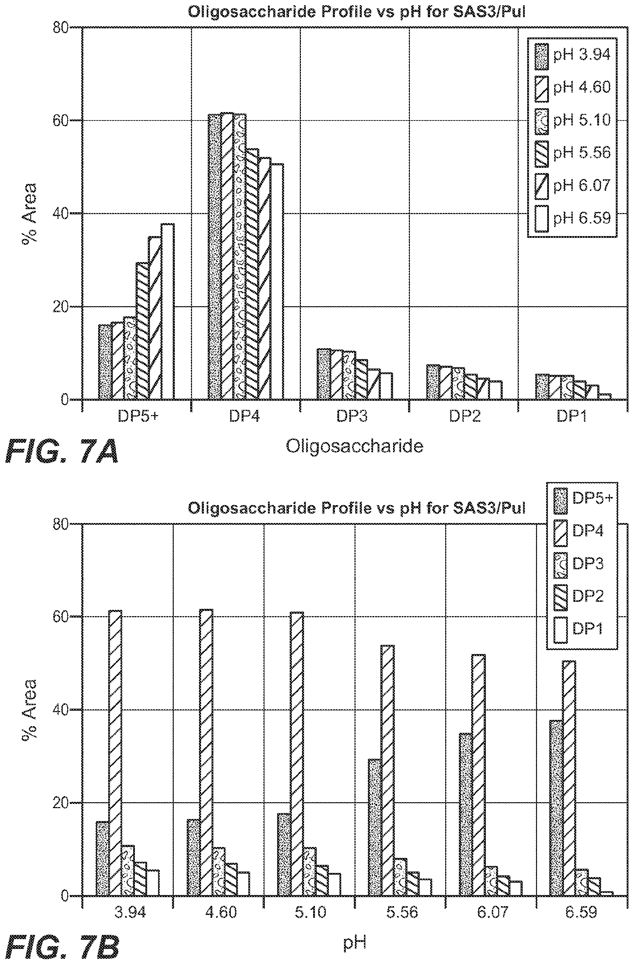

FIG. 7A and 7B depict the presence of oligosaccharides in saccharification reactions catalyzed by SAS3 and the pullulanase at various pH values (pH 3.94, 4.60, 5.10, 5.56, 6.07, and 6.59). The amounts of oligosaccharides are determined 21.5 hr after the reaction, when the DP4 levels reach the peak. Maltodextrin (Maltrin^{®} M040) is used herein as the saccharification substrate.

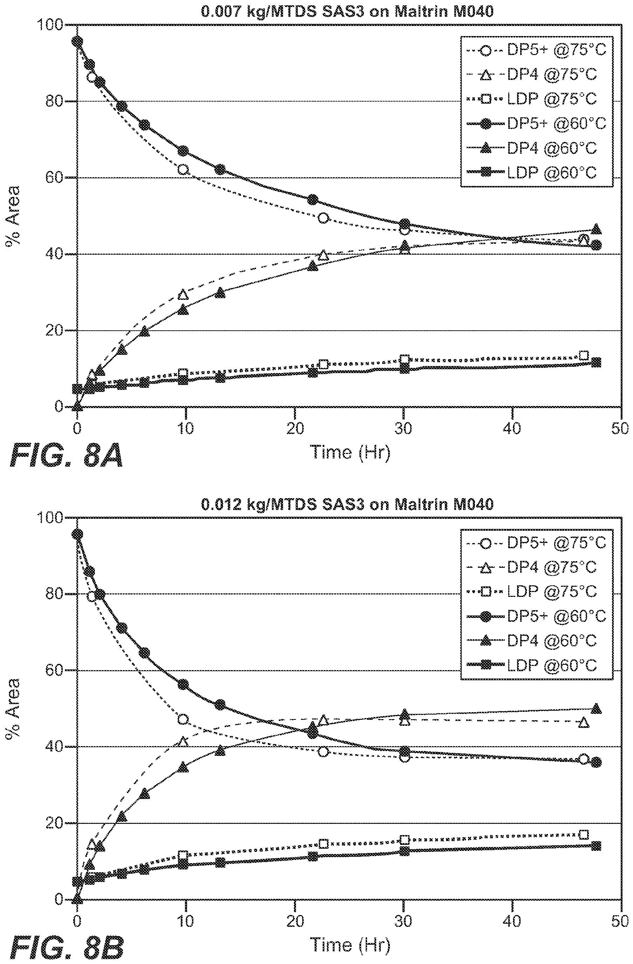

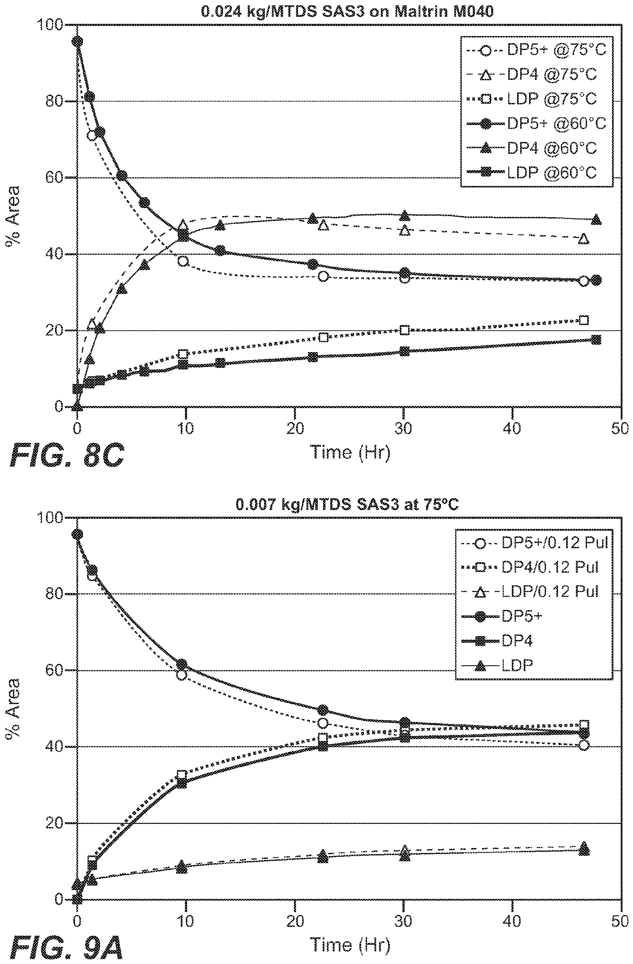

FIG. 8A depicts the comparison of oligosaccharide profiles in saccharification reactions catalyzed by 0.007 Kg/MTDS SAS3 at 60°C and 75°C. FIG. 8B depicts the comparison of oligosaccharide profiles in saccharification reactions catalyzed by 0.012 Kg/MTDS SAS3 at 60°C and 75°C. FIG. 8C depicts the comparison of oligosaccharide profiles in saccharification reactions catalyzed by 0.024 Kg/MTDS SAS3 at 60°C and 75°C. Maltodextrin (Maltrin^{®} M040) is used herein as the saccharification substrate.

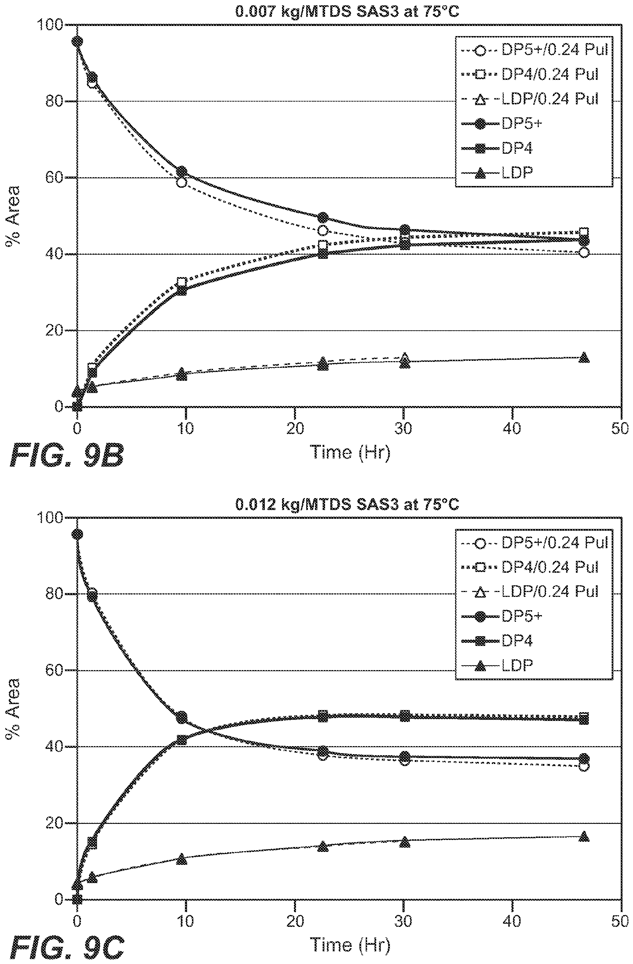

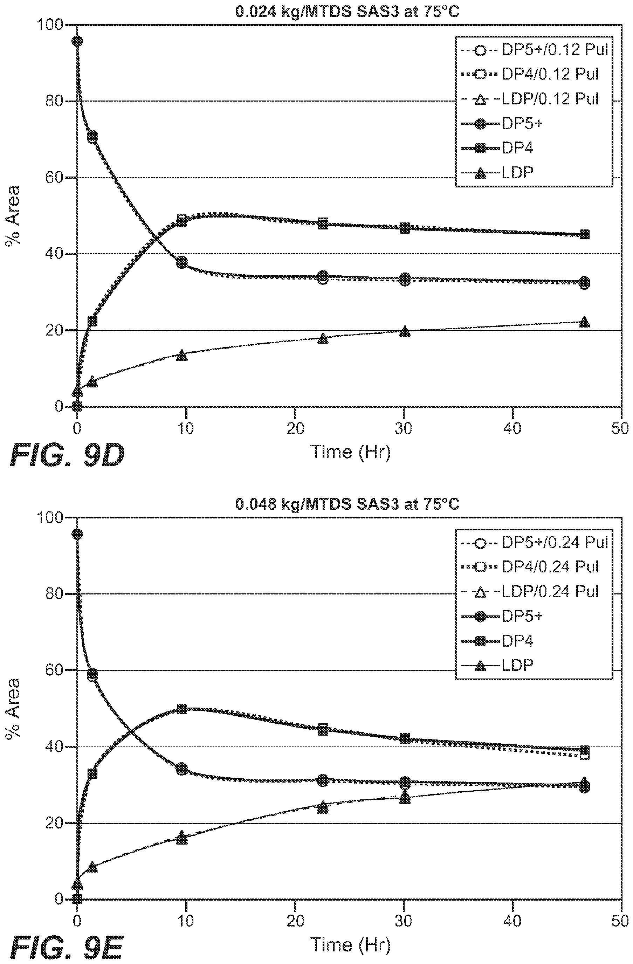

FIG. 9A depicts the comparison of oligosaccharide profiles in saccharification reactions catalyzed at 75°C by 0.007 Kg/MTDS SAS3 with or without supplementation of 0.12 Kg/MTDS pullulanase. FIG. 9B depicts the comparison of oligosaccharide profiles in saccharification reactions catalyzed at 75°C by 0.007 Kg/MTDS SAS3 with or without supplementation of 0.24 Kg/MTDS pullulanase. FIG. 9C depicts the comparison of oligosaccharide profiles in saccharification reactions catalyzed at 75°C by 0.012 Kg/MTDS SAS3 with or without supplementation of 0.24 Kg/MTDS pullulanase. FIG. 9D depicts the comparison of oligosaccharide profiles in saccharification reactions catalyzed at 75°C by 0.024 Kg/MTDS SAS3 with or without supplementation of 0.12 Kg/MTDS pullulanase. FIG. 9E depicts the comparison of oligosaccharide profiles in saccharification reactions catalyzed at 75°C by 0.048 Kg/MTDS SAS3 with or without supplementation of 0.24 Kg/MTDS pullulanase. Maltodextrin (Maltrin^{®} M040) is used herein as the saccharification substrate.

### DETAILED DESCRIPTION

A combination of a *Pseudomonas saccharophila* G4-forming amylase (PS4) variant and a pullulanase advantageously is capable of producing maltotetraose more efficiency from a starch-derived substrate, *e.g.*, liquefied starch or other source of maltodextrins. The enzyme combination can be used, therefore, to produce a maltotetraose syrup suitable for applications in food and pharmaceutical industries. PS4 may occasionally be referred to as SAS in the specification and figures. "PS4" and "SAS" are synonymous.

### 1. Definitions and abbreviations

In accordance with this detailed description, the following abbreviations and definitions apply. It should be noted that as used herein, the singular forms "a," "an," and "the" include plural referents unless the context clearly dictates otherwise. Thus, for example, reference to "an enzyme" includes a plurality of such enzymes, and reference to "the formulation" includes reference to one or more formulations and equivalents thereof known to those skilled in the art, and so forth.

Unless defined otherwise, all technical and scientific terms used herein have the same meaning as commonly understood by one of ordinary skill in the art. The following terms are provided below.

### 1.1. Definitions

"Amylase" means an enzyme that is, among other things, capable of catalyzing the degradation of starch. An endo-acting amylase activity cleaves α-D-(1→74) O-glycosidic linkages within the starch molecule in a random fashion. In contrast, an exo-acting amylolytic activity cleaves a starch molecule from the non-reducing end of the substrate. "Endo-acting amylase activity," "endo-activity," "endo-specific activity," and "endo-specificity" are synonymous, when the terms refer to PS4. The same is true for the corresponding terms for exo-activity.

"Pullulanase" refers to a specific kind of glucanase, an amylolytic endoenzyme, capable of catalyzing the hydrolysis of the α-1,6-glucosidic bonds. Pullulanases are able to degrade pullulan, which is regarded as a chain of maltotriose units linked by α-1,6-glucosidic bonds. Pullulanase is produced as an extracellular, cell surface-anchored lipoprotein by Gram-negative bacteria of the genus *Klebsiella.* Gram-positive bacteria, however, produce pullulanases as secreted protein. Pullulanase (E.C. 3.2.1.41) is also known as pullulan-6-glucanohydrolase, or simply as debranching enzyme.

A "variant" or "variants" refers to either polypeptides or nucleic acids. The term "variant" may be used interchangeably with the term "mutant." Variants include insertions, additions, deletions, substitutions, transversions, truncations, and/or inversions at one or more locations in the amino acid or nucleotide sequence, respectively. The phrases "variant polypeptide," and "variant enzyme" mean a protein or an enzyme that has an amino acid sequence that has been modified from the amino acid sequence of a wild-type protein or enzyme (parent). The variant polypeptides include a polypeptide having a certain percent, *e.g*., at least about 70%, about 75%, about 80%, about 85%, about 90%, about 95%, or about 99% (or any integer value between these numbers), of sequence identity with the parent enzyme. Variant polypeptides particularly may have a certain number of amino acid additions, deletions, or substitutions compared to the wild-type polypeptide. For example, PS4 variants may have 1 to 25, *e.g*., 1 to 5, 1 to 10, 1 to 15, or 1 to 20, amino acid additions deletions, or substitutions.

As used herein, "parent enzymes," "parent sequence," "parent polypeptide," "wild-type sequence," and "parent polypeptides" mean enzymes and polypeptides from which the variant polypeptides are based, *e.g*., the PS4 of SEQ ID NO: 1 or the pullulanase of SEQ ID NO: 6. A "parent nucleic acid" means a nucleic acid sequence encoding the parent polypeptide. A "wild-type" PS4 occurs naturally and includes naturally occurring allelic variants of the PS4 of SEQ ID NO: 1. A "wild-type" pullulanase occurs naturally and includes naturally occurring allelic variants of the pullulanase of SEQ ID NO: 6. The signal sequence of a "variant" may be the same or may differ from the wild-type enzyme. A variant may be expressed as a fusion protein containing a heterologous polypeptide. For example, the variant can comprise a signal peptide of another protein or a sequence designed to aid identification or purification of the expressed fusion protein, such as a His-Tag sequence.

"Variant nucleic acids" can include sequences that are complementary to sequences that are capable of hybridizing to the nucleotide sequences presented herein. For example, a variant sequence is complementary to sequences capable of hybridizing under stringent conditions, *e.g*., about 50°C and 0.2 × SSC (1× SSC = 0.15 MNaCl, 0.015 M sodium citrate, pH 7.0), to the nucleotide sequences presented herein. More particularly, the term variant encompasses sequences that are complementary to sequences that are capable of hybridizing under highly stringent conditions, *e.g*., about 65°C and 0.1× SSC, to the nucleotide sequences presented herein. The melting point (Tₘ) of a variant nucleic acid may be about 1°C, about 2°C, or about 3°C lower than the Tₘ of the wild-type nucleic acid. The variant nucleic acids include a polynucleotide having a certain percent, *e.g*., at least about 70%, about 75%, about 80%, about 85%, about 90%, about 95%, or about 99%, of sequence identity with the nucleic acid encoding the parent enzyme.

To describe the various variants, the following nomenclature will be adopted for ease of reference. Where the substitution includes a number and a letter, *e.g*., 141P, then this refers to {position according to the numbering system/substituted amino acid}. Accordingly, for example, the substitution of an amino acid to proline in position 141 is designated as 141P. Where the substitution includes a letter, a number, and a letter, *e.g*., A141P, then this refers to f original amino acid/position according to the numbering system/substituted amino acid}. Accordingly, for example, the substitution of alanine with proline in position 141 is designated as A141P.

Where two or more substitutions are possible at a particular position, this will be designated by contiguous letters, which may optionally be separated by slash marks "/", *e.g*., G303ED or G303E/D.

Sequence identity is determined using standard techniques known in the art (*see e.g.,* Smith and Waterman, Adv. Appl. Math. 2: 482 (1981); Needleman and Wunsch, J. Mol. Biol. 48: 443 (1970); Pearson and Lipman, Proc. Natl. Acad. Sci. USA 85: 2444 (1988); programs such as GAP, BESTHT, FASTA, and TFASTA in the Wisconsin Genetics Software Package (Genetics Computer Group, Madison, WI); and Devereux el al., Nucleic Acid Res., 12: 387-395 (1984)).

The "percent (%) nucleic acid sequence identity" or "percent (%) amino acid sequence identity" is defined as the percentage of nucleotide residues or amino acid residues in a candidate sequence that are identical with the nucleotide residues or amino acid residues of the starting sequence. The sequence identity can be measured over the entire length of the starting sequence.

"Sequence identity" is determined herein by the method of sequence alignment. For the purpose of the present disclosure, the alignment method is BLAST described by Altschul et al., (Altschul et al., J. Mol. Biol. 215: 403-410 (1990); and Karlin et al, Proc. Natl. Acad. Sci. USA 90: 5873-5787 (1993)). A particularly useful BLAST program is the WU-BLAST-2 program (*see* Altschul et al, Meth. Enzymol. 266: 460-480 (1996)). WU-BLAST-2 uses several search parameters, most of which are set to the default values. The adjustable parameters are set with the following values: overlap span =1, overlap fraction = 0.125, word threshold (T) = 11. The HSP S and HSP S2 parameters are dynamic values and are established by the program itself depending upon the composition of the particular sequence and composition of the particular database against which the sequence of interest is being searched. However, the values may be adjusted to increase sensitivity. A % amino acid sequence identity value is determined by the number of matching identical residues divided by the total number of residues of the "longer" sequence in the aligned region. The "longer" sequence is the one having the most actual residues in the aligned region (gaps introduced by WU-Blast-2 to maximize the alignment score are ignored).

As used herein, the term "expression" refers to the process by which a polypeptide is produced based on the nucleic acid sequence of a gene. The process includes both transcription and translation.

The term "isolated" refers to a material that is removed from the natural environment if it is naturally occurring.

A "purified" protein or enzyme refers to a protein that is at least partially purified to homogeneity. In some embodiments, a purified protein or enzyme is more than about 10% pure, optionally more than about 20% pure, and optionally more than about 30% pure, as determined by SDS-PAGE. Further aspects of the disclosure encompass the protein in a highly purified form (*i.e.*, more than about 40% pure, more than about 60% pure, more than about 80% pure, more than about 90% pure, more than about 95% pure, more than about 97% pure, and even more than about 99% pure), as determined by SDS-PAGE.

"Thermostable" or "thermostability" means the enzyme retains activity after exposure to elevated temperatures. The thermostability of an enzyme is measured by its half-life (t_{1/2}), where half of the enzyme activity is lost by the half-life. The half-life value is calculated under defined conditions by measuring the residual amylase activity. To determine the half-life of the enzyme, the sample is heated to the test temperature for 1-10 min, and activity is measured using a standard assay. The maltotetraohydrolase activity of a PS4 variant is measured with the Betamyl^{®} assay (Megazyme, Ireland), while the pullulanases activity is measured with the reducing sugars method. *See* U.S. Patent No. 5,736,375; Nelson N., "A Photometric Adaptation of the Somogyi Method for the Determination of Glucose," J. Biol. Chem. 153: 375-80 (1944); Somogyi M., "A New Reagent for the Determination of Sugars," J. Biol. Chem. 160: 61-68 (1945).

As used herein, "optimum pH" means the pH at which a PS4 variant supplemented with a pullulanase is the most active producing maltotetraose. The optimum pH is generally measured over a range of pH's.

As used herein, "amino acid sequence" is synonymous with the term "polypeptide" and/or the term "protein." In some instances, the term "amino acid sequence" is synonymous with the term "peptide"; in some instances, the term "amino acid sequence" is synonymous with the term "enzyme."

As used herein, "nucleotide sequence" or "nucleic acid sequence" refers to an oligonucleotide sequence or polynucleotide sequence and variants, homologues, fragments and derivatives thereof. The nucleotide sequence may be of genomic, synthetic or recombinant origin and may be double-stranded or single-stranded, whether representing the sense or anti-sense strand. As used herein, the term "nucleotide sequence" includes genomic DNA, cDNA, synthetic DNA, and RNA.

"Homologue" means an entity having a certain degree of identity or "homology" with the subject amino acid sequences and the subject nucleotide sequences. A "homologous sequence" includes a polynucleotide or a polypeptide having a certain percent, e.g., about 70%, about 75%, about 80%, about 85%, about 90%, about 95%, or about 99% (or any integer value in between), of sequence identity with another sequence. Percent identity means that, when aligned, that percentage of bases or amino acid residues are the same when comparing the two sequences. Amino acid sequences are not identical, where an amino acid is substituted, deleted, or added compared to the subject sequence. The percent sequence identity typically is measured with respect to the mature sequence of the subject protein, *i.e*., following removal of a signal sequence, for example. Typically, homologues will comprise the same active site residues as the subject amino acid sequence.

As used herein, "hybridization" includes the process by which a strand of nucleic acid joins with a complementary strand through base pairing, as well as the process of amplification as carried out in polymerase chain reaction (PCR) technologies. The variant nucleic acid may exist as single- or double-stranded DNA or RNA, an RNA/DNA heteroduplex or an RNA/DNA copolymer. As used herein, "copolymer" refers to a single nucleic acid strand that comprises both ribonucleotides and deoxyribonucleotides. The variant nucleic acid may be codon-optimized to further increase expression.

As used herein, a "synthetic" compound is produced by *in vitro* chemical or enzymatic synthesis. It includes, but is not limited to, variant nucleic acids made with optimal codon usage for host organisms, such as a yeast cell host or other expression hosts of choice.

As used herein, "transformed cell" includes cells, including both bacterial and fungal cells, which have been transformed by use of recombinant DNA techniques. Transformation typically occurs by insertion of one or more nucleotide sequences into a cell. The inserted nucleotide sequence may be a heterologous nucleotide sequence, *i.e.*, is a sequence that is not natural to the cell that is to be transformed, such as a fusion protein.

As used herein, "operably linked" means that the described components are in a relationship permitting them to function in their intended manner. For example, a regulatory sequence operably linked to a coding sequence is ligated in such a way that expression of the coding sequence is achieved under condition compatible with the control sequences.

As used herein, "biologically active" refers to a sequence having a similar structural, regulatory or biochemical function as the naturally occurring sequence, although not necessarily to the same degree.

As used herein the term "starch" refers to any material comprised of the complex polysaccharide carbohydrates of plants, such as corn, comprised of amylose and amylopectin with the formula (C₆H₁₀O₅)ₓ, where X can be any number. The term "granular starch" refers to raw, *i.e.,* uncooked starch, *e.g*., starch that has not been subject to gelatinization.

The term "liquefaction" refers to the stage in starch conversion in which gelatinized starch is hydrolyzed to give low molecular weight soluble dextrins, *i.e.* polysaccharides.

As used herein, the term "saccharification" refers to enzymatic conversion of starch, liquefied starch, or maltodextrins to saccharides, *e.g*., glucose.

The term "degree of polymerization" (DP) refers to the number (n) of anhydroglucopyranose units in a given saccharide. Examples of DPI are the monosaccharides glucose and fructose. Examples of DP2 are the disaccharides maltose and sucrose. An example of DP4, as used herein, is maltotetraose (G4). DP5+ refers to saccharides with a degree of polymerization of 5 or above, *e.g*., DP5, DP6, DP7, DP8, etc.

As used herein, the terms "dry solids content" or alternatively, "dissolved solids" (ds) refers to the total solids of a slurry or solution in a dry weight percent basis. The term "slurry" refers to an aqueous mixture containing insoluble solids.

As used herein "ethanologenic microorganism" refers to a microorganism with the ability to convert a sugar or oligosaccharide to ethanol.

As used herein, "contacting" or "admixing" refers to the placing of the respective enzyme(s) in sufficiently close proximity to the respective substrate to enable the enzyme(s) to convert the substrate to the end product. Those skilled in the art will recognize that mixing solutions of the enzyme with the respective substrates can effect contacting or admixing.

### 1.2. Abbreviations

The following abbreviations apply unless indicated otherwise:
- ADA: azodicarbonamide
- cDNA: complementary DNA
- CGTase: cyclodextrin glucanotransferase
- DE: dextrose equivalence
- DEAE: diethylamino ethanol
- DNA: deoxyribonucleic acid
- DPn: degree of polymerization with n glucose subunits
- ds: dry solids or dissolved solids
- EC: enzyme commission for enzyme classification
- FGSC: Fungal Genetics Stock Center
- G4: maltotetraose
- GRAS: generally recognized as safe
- HPLC: High Performance Liquid Chromatography
- Kg, kg: kilogram
- LDPs: low DPs (DP1, DP2, and DP3)
- MES: 2-(N-morpholino)ethanesulphonic acid
- MTDS: Metric tons dry solids
- mRNA: messenger ribonucleic acid
- PCR: polymerase chain reaction
- PEG: polyethyleneglycol
- ppm: parts per million
- PS4: *P. saccharophila* G4-forming amylase
- PUN: pullulanase unit
- RO water: Reverse osmosis water
- rpm: revolutions per minute
- RT-PCR: reverse transcriptase polymerase chain reaction
- SAS: *P. saccharophila* G4-forming amylase
- SAS3: *P. saccharophila* G4-forming amylase variant with the following substitutions: N33Y, D34N, G70D, G121F, G134R, A141P, Y146G, I157L, S161A, L178F, A179T, G223E, S229P, H307K, A309P, and S334P (SEQ ID NO: 3)
- SDS-PAGE: sodium dodecyl sulfate-polyacrylamide gel electrophoresis
- 1× SSC: 0.15 MNaCl, 0.015 M sodium citrate, pH 7.0
- t_{½}: half life
- Tₘ: melting temperature (°C) at which 50% of the subject protein is melted
- w/v: weight/volume
- w/w: weight/weight

### 2. Pseudomonas saccharophila alpha-amylase (PS4) variants

A nucleotide sequence encoding the wild-type PS4 has been determined. *See* Zhou et al., "Nucleotide sequence of the maltotetraohydrolase gene from Pseudomonas saccharophila," FEBS Lett. 255: 37-41 (1989); GenBank Acc. No. X16732. PS4 is expressed as a precursor protein with an N-terminal 21-residue signal peptide. The amino acid sequence of the PS4 precursor is set forth in SEQ ID NO: 1. The signal peptide is cleaved to form the mature form of PS4 containing 530 amino acid residues. The mature form has a catalytic domain at the N-terminus and a starch-binding domain at the C-terminus. The C-terminal starch binding domain of PS4 may be removed from the mature form PS4, leaving the catalytically active portion of PS4 having the amino acid sequence set forth in SEQ ID NO: 2.

An isolated and/or purified polypeptide comprising a variant PS4 is provided. In one embodiment, the variant PS4 is a mature form of the polypeptide, wherein the 21 amino acid leader sequence is cleaved, so that the N-terminus of the polypeptide begins at the aspartic acid (D) residue at position 22 of SEQ ID NO: 1. Variants of PS4 include a PS4 in which the C-terminal starch binding domain is removed. A representative amino acid sequence of a mature PS4 in which the starch biding domain is removed is set forth in SEQ ID NO: 2. Other PS4 variants include variants wherein between one and about 25 amino acid residues have been added or deleted with respect to wild-type PS4 or the PS4 of SEQ ID NO: 2. In one aspect, the PS4 variant has the amino acid sequence shown in SEQ ID NO: 2, wherein any number between one and about 25 amino acids have been substituted. In another aspect, a PS4 variant may have one or more amino acids added to the N-terminus of the PS4 of SEQ ID NO: 2, and the same variant may include between one and about 25 amino acids that have been substituted in the same sequence. A representative embodiment of these variants is set forth in SEQ ID NO: 3.

In another aspect, the PS4 variant has the sequence of wild-type PS4, wherein any number between one and about 25 amino acids have been substituted. Representative examples of PS4 variants having single amino acid substitutions are shown in TABLE 5. An example of a PS4 variant having combinations of amino acid substitutions is shown in TABLE 6. TABLE 6 depicts various amino acids that have been modified to form the sequence of SEQ ID NO: 3 (SAS3). In addition to the amino acid residue modifications listed in TABLES 5 - 6, additional specific PS4 residues that may be modified include A3, S44, A93, G103, V109, G172, A211, G265, N302, G313, and G342. PS4 variants may have various combinations of the amino acid substitutions disclosed herein. A process of using a PS4 variant may comprise the use of a single PS4 variant or a combination, or blend, of PS4 variants.

In one embodiment, the PS4 variant comprises an N-terminal methionine. The addition of a methionine at the amino terminus of the polypeptide may increase fermentation yields, for example.

A representative PS4 variant for formation of maltotetraose is SAS3, set forth in SEQ ID NO: 3. This variant has sixteen (16) substitutions that maintain or increase thermostability and pH stability compared to wild-type PS4: N33Y, D34N, G70D, G121F, G134R, A141P, Y146G, I157L, S161A, L178F, A179T, G223E, S229P, H307K, A309P, and S334P. In addition, this variant includes a methionine residue added to the N-terminus. In one embodiment, the PS4 variant comprises one or more of the following substitutions: N33Y, D34N, G70D, G121F, G134R, A141P, Y146G, I157L, S161A, L178F, A179T, S229P, H307K, A309P, or S334P. Additional amino acid substitutions can be made, for example: G121D, G223A, H272Q, G303E, and H307L.

Other particularly useful variants include those in which residues affecting substrate binding are substituted. PS4 residues involved in substrate binding include W66, I157, E160, S161, R196, W221, K222, H307, and W308. Substitutions of residues that affect substrate binding may affect the relative degree of endo- or exo-activity of the PS4 variant. A substitution that increases exo-activity, for example, advantageously promotes the formation of DP4 and DP3 saccharides. Representative examples of mutations affecting substrate binding include E160G, E160P, E160F, E160R, E160S, E160L, W66S, R196V, R196H, R196P, H307L, H307K, W221A, W308A, W308S, W308L, W308S, and K222T. These and additional variants of PS4 are described in U.S.S.N. 12/318,513, filed Dec. 30, 2008.

A contemplated PS4 variant may have at least about 70%, about 75%, about 80%, about 85%, about 90%, about 95%, about 98%, or about 99% sequence identity to the naturally occurring PS4 having an amino acid sequence of SEQ ID NO: 2. Moreover, the PS4 variant may display one or more altered properties compared to the PS4 having an amino acid sequence of SEQ ID NO: 2. Altered properties may include altered thermostability, altered stability at a given pH range, altered exo-alpha-amylase activity, or altered endo-alpha-amylase activity. The PS4 variant may display an improved thermostability and/or improved stability at a pH of about 5.0 to about 7.0 compared to the PS4 having an amino acid sequence of SEQ ID NO: 2. The PS4 variant may display an increased exo-alpha-amylase activity or an decreased endo-alpha-amylase activity compared to the PS4 having an amino acid sequence of SEQ ID NO: 2.

Nucleic acids encoding the polypeptides above also are provided. In one embodiment, a nucleic acid encoding a PS4 variant is a cDNA encoding the protein of SEQ ID NO: 2, comprising a codon modification that encodes a substituted amino acid. For example, the cDNA may have the corresponding sequence of the native mRNA, set forth in SEQ ID NO: 4. *See* GenBank Acc. No. X16732. As is well understood by one skilled in the art, the genetic code is degenerate, meaning that multiple codons in some cases may encode the same amino acid. Nucleic acids include genomic DNA, mRNA, or cDNA that encodes a PS4 variant.

### 2.1. PS4 variant characterization

Enzyme variants can be characterized by their nucleic acid and primary polypeptide sequences, by three-dimensional structural modeling, and/or by their specific activity. Additional characteristics of the PS4 variant include altered stability, optimal pH, oxidation stability, ratio of exo-amylase to endo-amylase activity, and thermostability, for example. Levels of expression and enzyme activity can be assessed using standard assays known to the artisan skilled in this field. In another aspect, variants demonstrate improved performance characteristics relative to the wild-type enzyme, such as improved stability at high temperatures, e.g., about 60-70°C. PS4 variants are advantageous for use for saccharification or other processes that require elevated temperatures. For example, a thermostable PS4 variant can degrade starch at temperatures of about 55°C to about 85°C or more.

An expression characteristic means an altered level of expression of the variant, when the variant is produced in a particular host cell. Expression generally relates to the amount of active variant that is recoverable from a fermentation broth using standard techniques known in this art over a given amount of time. Expression also can relate to the amount or rate of variant produced within the host cell or secreted by the host cell. Expression also can relate to the rate of translation of the mRNA encoding the variant enzyme.

A nucleic acid complementary to a nucleic acid encoding any of the PS4 variants set forth herein is provided. Additionally, a nucleic acid capable of hybridizing to the complement is provided. In another embodiment, the sequence for use in the methods and compositions described here is a synthetic sequence. It includes, but is not limited to, sequences made with optimal codon usage for expression in host organisms, such as yeast or bacteria.

### 3. Production of PS4 variants

The PS4 variants provided herein may be produced synthetically or through recombinant expression in a host cell, according to procedures well known in the art. The expressed PS4 variant optionally is isolated prior to use. The PS4 variant can be purified following expression. Leader or signal sequences can be cleaved. Methods of genetic modification and recombinant production of PS4 variants are described, for example, in U.S. Patent Nos. 7,371,552, 7,166,453; 6,890,572; and 6,667,065; and U.S. Published Application Nos. 2007/0141693; 2007/0072270; 2007/0020731; 2007/0020727; 2006/0073583; 2006/0019347; 2006/0018997; 2006/0008890; 2006/0008888; and 2005/0137111. Reference is made to the relevant teachings of these disclosures, including PS4-encoding polynucleotide sequences, primers, vectors, selection methods, host cells, purification and reconstitution of expressed PS4 variants, and characterization of PS4 variants, including useful buffers, pH ranges, Ca²⁺ concentrations, substrate concentrations and enzyme concentrations for enzymatic assays.

Suitable host cells include a Gram-positive bacterium selected from the group consisting of *Bacillus subtilis, B. licheniformis, B. lentus, B. brevis, B. stearothermophilus, B. alkalophilus, B. amyloliquefaciens, B. coagulans, B. circulans, B. lautus, B. thuringiensis, Streptomyces lividans,* or *S. murinus;* or a Gram-negative bacterium, wherein said Gram negative bacterium is *Escherichia coli* or a *Pseudomonas* species. In one embodiment, the host cell is *B. subtilis,* and the expressed protein is engineered to comprise a *B*. *subtilis* signal sequence, as set forth in further detail below.

A host cell can be genetically engineered to express an PS4 variant with an amino acid sequence having at least about 70%, about 75%, about 80%, about 85%, about 90%, about 95%, about 96%, about 97%, about 98% or about 99% identity with the wild-type PS4. The polynucleotide encoding a PS4 variant can have a nucleic acid sequence encoding the protein of SEQ ID NO: 2 or a nucleic acid sequence having at least about 70%, about 75%, about 80%, about 85%, about 90%, about 95%, about 96%, about 97%, about 98% or about 99% sequence identity with a nucleic acid encoding the protein of SEQ ID NO: 2. The nucleic acid sequence can have at least about 70%, about 75%, about 80%, about 85%, about 90%, about 95%, about 96%, about 97%, about 98% or about 99% sequence identity to the nucleic acid of SEQ ID NO: 4.

### 3.1. Vectors

A DNA construct comprising a nucleic acid encoding a PS4 variant is transferred to a host cell in an expression vector that comprises regulatory sequences operably linked to a PS4 encoding sequence. The vector may be any vector that can be integrated into a fungal host cell genome and replicated when introduced into a host cell. The FGSC Catalogue of Strains, University of Missouri, lists suitable vectors. Additional examples of suitable expression and/or integration vectors are provided in Sambrook et al., MOLECULAR CLONING: A LABORATORY MANUAL, 3rd ed., Cold Spring Harbor Laboratory Press, Cold Spring Harbor, New York (2001); Bennett et al., MORE GENE MANIPULATIONS IN FUNGI, Academic Press, San Diego (1991), pp. 396-428; and U.S. Patent No. 5,874,276. Exemplary vectors include pFB6, pBR322, PUC18, pUC100 and pENTR/D, pDON^{™}201, pDONR^{™}221, pENTR^{™}, pGEM^{®}3Z and pGEM^{®}4Z. Exemplary for use in bacterial cells include pBR322 and pUC19, which permit replication in *E. coli*, and pE194, for example, which permits replication in *Bacillus.*

A nucleic acid encoding a PS4 variant can be operably linked to a suitable promoter, which allows transcription in the host cell. The promoter may be derived from genes encoding proteins either homologous or heterologous to the host cell. Suitable non-limiting examples of promoters include *cbh1*, *cbh2*, *egl1*, and *egl2* promoters. The promoter can be one that is native to the host cell. For example, when *P. saccharophila* is the host, the promoter is a native *P. saccharophila* promoter. An "inducible promoter" is a promoter that is active under environmental or developmental regulation. The promoter can be one that is heterologous to the host cell.

The coding sequence can operably linked to a signal sequence. The DNA encoding the signal sequence may be the DNA sequence naturally associated with the PS4 nucleic acid to be expressed. The DNA encoding the signal sequence can be replaced with a nucleotide sequence encoding a signal sequence from a species other than *P. saccharophila.* The polynucleotide that encodes the signal sequence is immediately upstream and in-frame of the polynucleotide that encodes the polypeptide. The signal sequence may be selected from the same species as the host cell. In one non-limiting example, the signal sequence is a cyclodextrin glucanotransferase (CGTase; EC 2.4.1.19) signal sequence from *Bacillus* sp., and the PS4 variant is expressed in a *B*. *subtilis* host cell. A methionine residue may be added to the N-terminus of the signal sequence.

A signal sequence and a promoter sequence comprising a DNA construct or vector to be introduced into a fungal host cell can be derived from the same source. The expression vector can also include a termination sequence. The termination sequence and the promoter sequence are derived from the same source. The termination sequence is homologous to the host cell.

An expression vector can include a selectable marker. Examples of suitable selectable markers include those that confer resistance to antimicrobial agents, *e.g.*, hygromycin or phleomycin. Nutritional selective markers also are suitable and include *amdS*, *argB*, and *pyr4.* The selective marker is the *amdS* gene, which encodes the enzyme acetamidase; it allows transformed cells to grow on acetamide as a nitrogen source. The use of an *A. nidulans amdS* gene as a selective marker is described in Kelley et al., EMBO J. 4: 475-479 (1985) and Penttila et al., Gene 61: 155-164 (1987).

A suitable expression vector comprising a DNA construct with a polynucleotide encoding a PS4 variant may be any vector that is capable of replicating autonomously in a given host organism or integrating into the DNA of the host. The expression vector can be a plasmid. Two types of expression vectors for obtaining expression of genes are contemplated. The first expression vector comprises DNA sequences in which the promoter, PS4 coding region, and terminator all originate from the gene to be expressed. Gene truncation can be obtained by deleting undesired DNA sequences, *e.g*., DNA encoding the C-terminal starch binding domain, to leave the domain to be expressed under control of its own transcriptional and translational regulatory sequences. The second type of expression vector is preassembled and contains sequences required for high-level transcription and a selectable marker. The coding region for a PS4 gene or part thereof can be inserted into this general-purpose expression vector, such that it is under the transcriptional control of the expression construct promoter and terminator sequences. Genes or part thereof can be inserted downstream of the strong *cbh1* promoter. C-terminal truncation of expressed PS4 variant is contemplated. For example, C-terminal truncation of alpha-amylases is described in Ohdan et al., Applied and Environ. Microbiol. 65: 4652-4658 (1999).

### 3.2. Transformation, expression and culture of host cells

Introduction of a DNA construct or vector into a host cell includes techniques such as transformation; electroporation; nuclear microinjection; transduction; transfection, *e.g*., lipofection mediated and DEAE-Dextrin mediated transfection; incubation with calcium phosphate DNA precipitate; high velocity bombardment with DNA-coated microprojectiles; and protoplast fusion. General transformation techniques are known in the art. *See, e.g*., Ausubel *et al.* (1987), *supra*, chapter 9; Sambrook *et al.* (2001), *supra*; and Campbell et al., Curr. Genet. 16: 53-56 (1989). The expression of heterologous protein in *Trichoderma* is described, for example, in U.S. Patent No. 6,022,725; U.S. Patent No. 6,268,328; Harkki et al., Enzyme Microb. Technol. 13: 227-233 (1991); Harkki et al., BioTechnol. 7: 596-603 (1989); EP 244,234; and EP 215,594. Genetically stable transformants can be constructed with vector systems whereby the nucleic acid encoding a PS4 variant is stably integrated into a host cell chromosome. Transformants are then purified by known techniques.

In one non-limiting example, stable transformants including an *amdS* marker are distinguished from unstable transformants by their faster growth rate and the formation of circular colonies with a smooth, rather than ragged outline on solid culture medium containing acetamide. Additionally, in some cases a further test of stability is conducted by growing the transformants on solid non-selective medium, e.g., a medium that lacks acetamide, harvesting spores from this culture medium and determining the percentage of these spores that subsequently germinate and grow on selective medium containing acetamide. Other methods known in the art may be used to select transformants.

### 3.3. Identification of PS4 activity

To evaluate the expression of a PS4 variant in a host cell, assays can measure the expressed protein, corresponding mRNA, or alpha-amylase activity. For example, suitable assays include Northern and Southern blotting, RT-PCR (reverse transcriptase polymerase chain reaction), and *in situ* hybridization, using an appropriately labeled hybridizing probe. Suitable assays also include measuring PS4 activity in a sample. Suitable assays of the exo-activity of the PS4 variant include, but are not limited to, the Betamyl^{®} assay (Megazyme, Ireland). Suitable assays of the endo-activity of the PS4 variant include, but are not limited to, the Phadebas blue assay (Magle Life Sciences). Assays also include HPLC analysis of saccharide syrup prepared in the presence of the PS4 variant. HPLC, for example, can be used to measure amylase activity by separating DP4 saccharides from other saccharides in the reaction mixture.

### 3.4. Methods for purifying PS4

In general, a PS4 variant produced in cell culture is secreted into the medium and may be purified or isolated, e.g., by removing unwanted components from the cell culture medium. In some cases, a PS4 variant may be recovered from a cell lysate. In such cases, the enzyme is purified from the cells in which it was produced using techniques routinely employed by those of skill in the art. Examples include, but are not limited to, affinity chromatography, ion-exchange chromatographic methods, including high resolution ion-exchange including HPLC on sulfonated styrene-divinylbenzene ion-exchange resin, hydrophobic interaction chromatography, two-phase partitioning, ethanol precipitation, reverse phase HPLC, chromatography on silica or on a cation-exchange resin, such as DEAE, chromatofocusing, SDS-PAGE, ammonium sulfate precipitation, gel permeation chromatography (GPC), and gel filtration (size exclusion chromatography) using Sephadex G-75, for example.

### 4. Pullulanases

Pullulanases (E.C. 3.2.1.41) are debranching enzymes characterized by their ability to hydrolyze the α-1,6-glycosidic bonds in, for example, amylopectin and pullulan. Pullulanases have been found useful in various industrial applications, particularly in the food and beverage industries. Pullulanases are starch debranching enzymes and are effective in the debranching of starch hydrolysates (useful in conditioning dough), the debranching of beta-limit dextrans (useful in the brewing of beer and ales), and in the production of sugar syrups from starch-containing materials, such as corn, potato, wheat, manioc, and rice.

Pullulanase activity may be measured with the reducing sugars method as described in U.S. Patent No. 5,736,375. *See also*, Nelson N., "A Photometric Adaptation of the Somogyi Method for the Determination of Glucose," J. Biol. Chem. 153: 375-80 (1944); Somogyi M., "A New Reagent for the Determination of Sugars," J. Biol. Chem. 160: 61-68 (1945).

Representative pullulanases include those from the genus *Bacillus,* particularly the pullulanase from *Bacillus amyloderamificans* as disclosed in U.S. Patent No. 4,560,651, the pullulanase disclosed as SEQ ID NO: 2 in WO 01/051620, the pullulanase from *Bacillus deramificans* disclosed as SEQ ID NO: 4 in WO 01/051620, and the pullulanase from *Bacillus acidopullulyticus* disclosed as SEQ ID NO: 6 in WO 01/051620. *See also*, Kelly et al., "Molecular Genetic analysis of the Pullulanase B Gene of Bacillus acidopullulyticus," FEMS Microbiol. Lett. 115: 97-106 (1994).

Additionally, the pullulanase from *Bacillus deramificans*, having an amino acid sequence of SEQ ID NO: 6, and its variants thereof may be used in the presently described application. The production of the pullulanase having an amino acid sequence of SEQ ID NO: 6 has been described in U.S. Patent Nos. 5,736,375 and 7,399,623.

The pullulanase useful in the present disclosure may be a variant of a naturally occurring enzyme. The variant may have an amino acid sequence having about 70%, about 75%, about 80%, about 85%, about 90%, about 95%, about 98%, or about 99% identity to SEQ ID NO: 6. The variant pullulanase may display one or more altered properties compared to the pullulanase with an amino acid sequence of SEQ ID NO: 6. The altered properties may include: improved thermostability, pH dependent activity, specific activity, substrate specificity, or any combination thereof.

Suitable commercially available pullulanase products include PROMOZYME D, PROMOZYMETM D2 (Novozymes A/S), OPTIMAX L-300 (Danisco US Inc., Genencor Division), and AMANO 8 (Amano, Japan).

To produce the pullulanase, a polynucleotide encoding the *Bacillus deramificans* pullulanase, *e.g*., SEQ ID NO: 7 or its variants thereof, may be cloned in an expression vector and introduced into a microorganism. The microorganism may be *B. subtilis*, *B. licheniformis, B. lentus, B. brevis, B. stearothermophilus, B. alkalophilus, B. amyloliquefaciens*, *B. coagulans*, *B. circulans*, *B. lautus*, *and B. thuringiensis.* Typically, the *Bacillus* cell is modified a *B. licheniformis* strain that comprises an altered Carlsberg protease gene and/or an altered endo Glu C protease gene. *See* Jacobs et al., Nucleic Acid Res. 13:8913-26 (1985); *see also* Kakudo et al., J. Bio. Chem. 267:23782-88 (1992). The protease activity is essentially eliminated in the modified *B. licheniformis,* so that the production of the pullulanase is greatly improved. *See* U.S. Patent No. 7,399,623.

To produce a maltotetraose syrup from liquefied starch or other source of maltodextrins, the pullulanase may be added, together with a PS4 variant, in an effective amount of about 0.10, about 0.20, about 0.30, about 0.40, or about 0.50 Kg/MTDS.

### 5. Uses of PS4 variants and pullulanase in maltotetraose production

The desirability of using a particular PS4 variant will depend on the overall properties displayed by the PS4 variant relative to the requirements of a particular application. For example, PS4 variants useful for a starch conversion process may have substantial endo-amylase activity compared to wild-type PS4, and/or have a lower exo- to endo-amylase activity compared to wild-type PS4. Such PS4 variants may be particularly useful in a process where internal cleavage of complex branching saccharides in useful in lowering the viscosity of the substrate. Useful PS4 variants include those with more or less exo-amylase activity than the wild-type PS4, depending on the application. Compositions may include one or a combination of PS4 variants, each of which may display a different set of properties.

### 5.1. Preparation of starch substrates

Methods to prepare starch substrates are well known in the art. For example, a useful starch substrate may be obtained from tubers, roots, stems, legumes, cereals or whole grain. More specifically, the granular starch comes from plants that produce high amounts of starch. For example, granular starch may be obtained from corns, cobs, wheat, barley, rye, milo, sago, cassava, tapioca, sorghum, rice, peas, bean, banana, or potatoes. Corn contains about 60-68% starch; barley contains about 55-65% starch; millet contains about 75-80% starch; wheat contains about 60-65% starch; and polished rice contains 70-72% starch. Specifically contemplated starch substrates are cornstarch, wheat starch, and barley starch. The starch from a grain may be ground or whole and includes corn solids, such as kernels, bran and/or cobs. The starch may be highly refined raw starch or feedstock from starch refinery processes. Various starches also are commercially available. For example, cornstarch is available from Cerestar, Sigma, and Katayama Chemical Industry Co. (Japan); wheat starch is available from Sigma; sweet potato starch is available from Wako Pure Chemical Industry Co. (Japan); and potato starch is available from Nakaari Chemical Pharmaceutical Co. (Japan).

Maltodextrins are useful as starch substrates in embodiments of the present disclosure. Maltodextrins comprise starch hydrolysis products having about 20 or fewer dextrose (glucose) units. Typical commercial maltodextrins contain mixtures of polysaccharides including from about three to about nineteen linked dextrose units. Maltodextrins are defined by the FDA as products having a dextrose equivalence (DE) of less than 20. They are generally recognized as safe (GRAS) food ingredients for human consumption. Dextrose equivalence (DE) is a measure of reducing power compared to a dextrose (glucose) standard of 100. The higher the DE, the greater the extent of starch depolymerization, resulting in a smaller average polymer (polysaccharide) size, and the greater the sweetness. A particularly useful maltodextrin is MALTRIN^{®} M040 obtained from cornstarch, available from Grain Processing Corp. (Muscatine, Iowa): DE 4.0-7.0; bulk density 0.51 g/cc; measured water content 6.38% by weight.

The starch substrate can be a crude starch from milled whole grain, which contains non-starch fractions, e.g., germ residues and fibers. Milling may comprise either wet milling or dry milling. In wet milling, whole grain is soaked in water or dilute acid to separate the grain into its component parts, *e.g*., starch, protein, germ, oil, kernel fibers. Wet milling efficiently separates the germ and meal (*i.e*., starch granules and protein) and is especially suitable for production of syrups. In dry milling, whole kernels are ground into a fine powder and processed without fractionating the grain into its component parts. Dry milled grain thus will comprise significant amounts of non-starch carbohydrate compounds, in addition to starch. Alternatively, the starch to be processed may be a highly refined starch quality, for example, at least 90%, at least 95%, at least 97%, or at least 99.5% pure.

### 5.2. Saccharification of liquefied starch or maltodextrins

As used herein, the term "liquefaction" or "liquefy" means a process by which starch is converted to less viscous and shorter chain dextrins. This process involves gelatinization of starch simultaneously with or followed by the addition of a PS4 variant and a pullulanase. A thermostable PS4 variant is typically used for this application. Additional liquefaction-inducing enzymes optionally may be added.

In some embodiments, the starch or maltodextrin substrate prepared as described above is slurried with water. The starch or maltodextrin slurry may contain starch as a weight percent of dry solids of about 10-55%, about 20-45%, about 30-45%, about 30-40%, or typically about 30-35%. Alpha-amylases, *e.g*., bacterial alpha-amylases, including *Bacillus* alpha-amylases, may be supplied, at about 1500 units per kg dry matter of starch, for example. To optimize alpha-amylase stability and activity, the pH of the slurry may be adjusted to the optimal pH for the alpha-amylase. Other alpha-amylases may be added and may require different optimal conditions. Bacterial alpha-amylase remaining in the slurry following liquefaction may be deactivated by lowering pH in a subsequent reaction step or by removing calcium from the slurry.

The slurry of starch may be pumped continuously through a jet cooker, which is steam heated from about 85°C to up to 105°C. Gelatinization occurs very rapidly under these conditions, and the enzymatic activity, combined with the significant shear forces, begins the hydrolysis of the starch substrate. The residence time in the jet cooker is very brief. The partly gelatinized starch may be passed into a series of holding tubes maintained at about 85-105°C and held for about 5 min. to complete the gelatinization process. These tanks may contain baffles to discourage back mixing. As used herein, the term "secondary liquefaction" refers the liquefaction step subsequent to primary liquefaction, when the slurry is allowed to cool to room temperature. This cooling step can be about 30 minutes to about 180 minutes, e.g., about 90 minutes to 120 minutes.

A PS4 variant and a pullulanase can be added to the liquefied starch obtained by the process above or to a maltodextrin slurry to produce maltotetraose. The PS4 variant may be added at about 0.005, about 0.01, about 0.02, about 0.03, about 0.04, about 0.05, about 0.06, about 0.07, about 0.08, about 0.09, about 0.1, about 0.2, about 0.3, about 0.4, about 0.5, about 0.6, about 0.7, about 0.8, about 0.9, or about 1.0 kg/MTDS. 1 kg/MTDS = 0.1 % by weight dissolved solids. In one embodiment, the PS4 variant is added in a range of about 0.01 to about 0.05 kg/MTDS. The pullulanase may be supplemented in an amount that is about 5, about 10, about 15, about 20, about 25, about 30, about 35, about 40, about 45, or about 50 times of the amount of the PS4 variant in kg/MTDS.

In yet another embodiment, either the PS4 variant or the pullulanase, or both enzymes are immobilized, and the liquefied starch or maltodextrin substrate is passed over the immobilized enzyme and converted to product in a continuous reaction.

The saccharification catalyzed by a PS4 variant and a pullulanase may be carried at a pH in the range of 3.90 to 6.60. Typically, the pH suitable for maltotetraose production may be in the range of 3.90 to 5.10.

The combination of a PS4 variant and a pullulanase is capable of producing an increased amount of maltotetraose, *e.g*., about 20% more than using the PS4 variant alone. As a result of saccharification reaction catalyzed by a PS4 variant supplemented with a pullulanase, a saccharide syrup can be formed comprising at least about 40%, about 45%, about 50%, about 55%, or about 60% by weight maltotetraose based on dissolved solids, *i.e.* based on total saccharide content.

The production of maltotetraose may further comprise contacting the liquefied starch or other source of maltodextrins with an isoamylase, a protease, a cellulase, a hemicellulase, a lipase, a cutinase, or any combination thereof.

### EXAMPLES

Unless otherwise indicated, all percentages are expressed in weight percent. HPLC chromatography was employed to determine distribution of saccharide products.

### Example 1: Materials and methods

### 1.1. Maltodextrin

Samples of Maltrin^{®} M040 (Maltodextrin) were obtained from Grain Processing Corporation (Muscatine, Iowa). DE was reported from the manufacturer as 4.0-7.0. The measurement of humidity gave an average of 6.38% by weight (water content). The humidity of the Maltodextrin sample was taken in consideration when preparing the 32% DS slurry of for experiments.

### 1.2. Liquefact

Liquefact samples were obtained from Badger State Ethanol LLC, a dry-mill ethanol production facility in Monroe, Wisconsin. The liquefact appeared as a yellow/green mash. The reported pH was around 5.8, while the actual pH was approximately 6.1. The liquefact also had a reported DS of 32-35% and a reported DE value between 10 and 15.

### 1.3. Preparation of SAS3

The genetically modified *Pseudomonas saccharophila* amylase (SAS3; SEQ ID NO: 3) was expressed in *Bacillus licheniformis* using an IPTG-inducible pET expression vector according to known methods. After purification, filtration, and concentration, crystals of the enzyme were harvested and dried at 37°C. Solutions of the enzyme were made by dissolving a known amount of enzyme in 50 mM sodium citrate buffer, which has a pH of 6.5 and was pre-warmed at 60°C. The stock solution was prepared to have a concentration of 3 mg/mL SAS3.

### 1.4. Preparation of pullulanase

The production of pullulanase from *B. deramificans* has been described in U.S. Patent No. 6,074,854. The particular *Bacillus deramificans* strain used for pullulanase production is *B. deramificans* T 89.117D, which has been deposited in the collection called the BELGIAN COORDINATED COLLECTIONS OF MICROORGANISMS (LMG culture collection) under number LMG P-13056. The *B. deramificans* T89.117D strain produces an extracellular pullulanase having an amino acid sequence of SEQ ID NO: 6.

The liquid culture of *Bacillus deramificans* T 89.117D was carried out at a temperature of 37°C with effective aeration. After 68 hours of culture, the pullulanase and the cell biomass were separated by centrifugation (5000 rpm for 30 minutes, BECKMAN JA-10). The pullulanase was then concentrated by ultrafiltration (AMICON S10 Y10 membrane) to obtain a concentrated aqueous solution of pullulanase.

The enzymatic activity of the solution obtained was measured throughout the isolation / purification steps using methods that have been described in U.S. Patent No. 6,074,854. One enzymatic unit of pullulanase (PUN) is defined as the amount of enzyme that catalyses the release of reducing sugars at a rate of 1 µmol glucose equivalent per minute, at a pH of 4.5, at a temperature of 60°C and in the presence of pullulan as the substrate.

In order to further purify the pullulanase, the aqueous concentrated solution of pullulanase was diafiltered by 6 portions of 500 ml of an aqueous solution of 9 g/l of NaCl, and the pH of the aqueous solution thus obtained was adjusted to about pH 3.5 by addition of 25% (v/v) strength HCl at room temperature. The diafiltration comprises mixing the pullulanase solution with the NaCl solution and then subjecting the solution obtained to ultrafiltration. The precipitate obtained was removed by centrifugation (5000 rpm for 30 minutes, BECKMAN JA-10), and the supernatant from the centrifugation was collected. The pH of this supernatant was adjusted to pH 6.0 by addition of 5 M NaOH. The precipitate obtained was removed by centrifugation. The supernatant from the centrifugation was collected and was heated at 55°C for 15 minutes. The precipitate formed was removed again by centrifugation (5000 rpm for 30 minutes, BECKMAN JA-10). The supernatant from the centrifugation was collected. Acetone was added to this supernatant to a final concentration of 60% (v/v), and the suspension formed was brought to 4°C over a period of 2 hours. The precipitate formed at 4°C was dissolved in a buffer of 20 mM MES (2-(N-morpholino)ethanesulphonic acid) and 1 mM CaCl₂ (pH 6.0). This pullulanase solution was called solution A.

This solution A was concentrated again by ion exchange chromatography in order to purify the pullulanase. A column of about 20 ml internal volume, sold under the trade name S-SEPHAROSE^{®} HP HI LOAD 16/10, was first equilibrated with a buffer of 50 mM sodium acetate and 100 mM NaCl (pH 4.0) at a flow rate of 5 ml/minute. Solution A was diluted 10 times in the acetate buffer and 15 ml of this dilute solution are deposited on the column. An isocratic phase was ensured by elution of 80 ml of acetate buffer (100 mM NaCl), followed by elution by 200 ml of 50 mM acetate buffer (pH = 4.0) containing a linear gradient of NaCl (100-500 mM). The pullulanase activity was measured in each fraction. The most active fractions were combined into a solution called B (12 ml containing 0.025 mg/ml of proteins and having a pullulanase activity of 0.7 PUN/ml). Starting from solution B, precipitation was effected with acetone at a final concentration of 80% (v/v). The precipitate obtained was dissolved in a volume of 0.6 ml of buffer comprising 20 mM MES and 1 mM CaCl₂ (pH 6.0). This pullulanase solution was called solution C, which had a protein content of 0.4 mg/ml, an enzymatic activity of 12 PUN/ml and a specific activity of 30 PUN/mg. Solution C has been shown to be suitable for most applications.

Alternatively, the pullulanase can be obtained through recombinant DNA technology. For this, a desired pullulanase gene, *e.g*., SEQ ID NO: 5 or 7, may be cloned in a proper vector, operably linked downstream of a promoter, and introduced into a *B. licheniformis* host cell. The *B. licheniformis* host may have a deletion of the Carlsburg protease and/or a deletion of the endo Glu C protease. The resulting recombinant strain can be cultured under conditions suitable for expression of the pullulanase gene and secretion of the expressed pullulanase. *See* U.S. Patent No. 7,449,320.

### 1.5. HPLC analysis

The distribution of saccharide products was determined by HPLC at 60°C. Samples were analyzed with a Agilent 1200 series (Agilent Technologies, Palo Alto, CA) using a Aminex HPX-87H 300 7.8 mm column with guard. The eluent was 0.01 N sulfuric acid. The flow of the eluent was adjusted to 0.6 ml/min. Components of a saccharification reaction were determined with a Refracted Index Detector at 55°C by injecting 0.02 mL of sample (2% solution in 0.01 N sulfuric acid of incubation mixture). The sample was run either 15 or 24 minutes. Four different concentrations of commercial standards of DP1 (glucose), DP2 (maltose), DP3 (maltotriose), DP4 (maltotetraose), DP5 (maltopentaose), and DP6 (maltohexaose) were used to calibrate the RI response. Signals were processed using ChemStation for LC 3D software (Agilent Technologies, Palo Alto, CA).

### Example 2: Effect of SAS3 on the production of maltotetraose syrup from liquefact

Liquefact samples were prepared by weighing 4.0 ± 0.05 g of the crude liquefact in 18 × 150 mm glass test tubes. The pH was left unadjusted. SAS3 was inoculated to the slurry at a dose of 0.048 Kg/MTDS. Test tubes were stirred, capped with a plastic cover, and incubated in a water bath at 60°C. Approximately 50-100 µl sample was taken from each test tube at various time points (0, 3, 24.2, 70, and 94 hours). Each removed sample was suspended in 1 mL 0.01 N sulfuric acid in an Eppendorf tube. The suspension was centrifuged at 14,000 rpm for 2 minutes, and the supernatant was filtered through a 0.45 µm syringe filter. The filtrate was subject to HPLC analysis for oligosaccharide contents as described in Example 1.5 *supra.* The experiment was performed in triplicate, and the average values were presented in FIGS. 1A, 1B, and 2.

The data confirmed that SAS3 is able to use the liquefact as a substrate to produce maltotetraose syrup. *See* FIGS. 1A and 1B. Under the conditions tested, maltotetraose may represent up to 40% of total oligosaccharides in the supernatant liquor. *See* FIG. 2. Additionally, it was observed that a longer incubation time may result in a decreased level of DP4 and concomitantly an increased level of low DPs (LDPs; DP1, DP2, and DP3 collectively). An incubation time of 14-18 hr appears optimal for maltotetraose production under the conditions tested. Furthermore, there was a dramatic reduction of high DPs (DP5+) immediately after the addition of SAS3. After about 10 hours, however, the amount of DP5+ remained relatively constant, representing approximately 25% of total oligosaccharides in the supernatant.

### Example 3: Effect of pullulanase on the SAS3-mediated production of maltotetraose syrup from liquefact

Although SAS3 is able to convert a liquefact substrate to a saccharide syrup containing about 40% maltotetraose, a significant amount of DP5+, approximately 25%, remains intact. The DP5+ represented oligosaccharides resistant to SAS3-catalyzed hydrolysis. The DP5+ species likely included glucose units linked through α-1,6-glycosidic bonds, which SAS3 is unable to hydrolyze. The pullulanase, on the other hand, is known for its ability to catalyze the hydrolysis of α-1,6-glycosidic bonds. To maximize maltotetraose production, the effect of supplementing a pullulanase to SAS3 in the saccharification was further investigated.

Sample preparation and analysis were similar to Example 2, except that SAS3 was dosed at 0.046 Kg/MTDS, and pullulanase was dosed at 0.24 Kg/MTDS. The results were presented in FIGs. 3A, 3B, and 4. Overall, the addition of a pullulanase to SAS3 resulted in increased maltotetraose production. *See* FIG. 4. Under the conditions tested, *i.e.,* 0.046 Kg/MTDS SAS3 and 0.24 Kg/MTDS pullulanase, maltotetraose represented up to 50% of oligosaccharides in the supernatant liquor. It reflected a 20% increase of maltotetraose production compared to saccharification catalyzed by SAS3 alone. *See* FIGs. 3A and 3B. The increased level of DP4 was concomitant with a substantially decreased level of DP5+. *See* FIG. 4. This observation suggested that a significant amount of DP5+ becomes amenable to SAS3-mediated hydrolysis after the pullulanase acts on the α-1,6-glycosidic linkages. Similar to what was observed in Example 2, it appears that a longer incubation time may result in the hydrolysis of DP4 into low DPs. An incubation time of 15-28 hr appears optimal for maltotetraose production under the conditions tested.

### Example 4: Comparison of maltotetraose production by adding varying amounts of SAS3 and a fix amount of pullulanase

To optimize the maltotetraose production by the combination of SAS3 and a pullulanase, various combinations of SAS3 and a pullulanase were added to the reaction to determine the optimal ratio between the two enzymes. Briefly, maltodextrin was dissolved in tap water to reach a 32% DS value. The pH was adjusted to approximately 5.6 with 0.1 M sodium carbonate solution. A slurry of 4 g was added to a 18 × 150 mm glass test tube and inoculated with SAS3 at a concentration of 0.007, 0.012, or 0.024 Kg/MTDS. The pullulanase dosage was fixed at 0.24 Kg/MTDS. Test tubes were stirred, capped with plastic covers, and placed in a water bath at 60°C. Approximately a 20 µl sample was taken from each test tube at various time points and subject to HPLC analysis as described in Example 2.

The results were presented in FIGS. 5A, 5B, 5C, and 6. The combination of 0.012 Kg/MTDS SAS3 and 0.24 Kg/MTDS pullulanase was the most effective in producing maltotetraose. *See* FIG. 5B. Compared to the maltotetraose production by 0.012 Kg/MTDS SAS3 alone, the supplementation of 0.24 Kg/MTDS pullulanase resulted in an increase of approximately 20% maltotetraose, *i.e.*, improving from about 50% maltotetraose to about 60% maltotetraose in the total saccharides. Additionally, the effect of the pullulanase upon maltotetraose product is evidenced more prominently after relatively long incubation periods, *e.g*., more than 30 hr.

### Example 5: Comparison of maltotetraose production by the combination of SAS3 and pullulanase at various pHs.

To further optimize maltotetraose production by the combination of SAS3 and a pullulanase, saccharification reactions were carried out at various pH values. Briefly, maltodextrin was dissolved in tap water to reach a 32% DS value. The pH was adjusted to 3.94, 4.60, 5.10, 5.56, 6.07, and 6.59, using either 0.1 M solution of sulfuric acid or 0.1 M solution of sodium carbonate. SAS3 was dosed at 0.048 Kg/MTDS, and the pullulanase was dosed at 0.24 Kg/MTDS. The reactions were carried as described in Example 4. At various time intervals (2.5, 21.5, and 94.5 hr), 0.02 mL sample was removed and suspended in 0.01 N sulfuric acid. The oligosaccharide profile for each sample as analyzed by a HPLC method as described in Example 2.

The data are presented in Table 1. Additionally, FIGS. 7A and 7B show the oligosaccharide profile at 21.5 hr, when the DP4 yield was the highest across tested pHs. Overall, the data suggest that a maximum DP4 yield occurs at pHs below 5.5. Similarly, the DP5+ level is the lowest. Formation of low DPs (DP1, DP2, and DP3) is favored at lower pHs, while reactions carried at higher pHs yielded the least amount of low DPs. Furthermore, the hydrolysis of DP5+ became less efficient at pH values higher than 5.1.

**Table 1.**

| | **pH** | **SAS3 Kq/MTDS** | **Pul Kg/MTDS** | **DP5+** | **DP4** | **DP3** | **DP2** | **DP1** |
|---|---|---|---|---|---|---|---|---|
| **2.5 hr** | 3.94 | 0.048 | 0.24 | 67.756 | 25.318 | 3.238 | 2.320 | 1.367 |
| | 4.60 | 0.048 | 0.24 | 52.442 | 36.995 | 5.106 | 3.401 | 2.056 |
| | 5.10 | 0.048 | 0.24 | 54.266 | 35.943 | 4.719 | 3.132 | 1.940 |
| | 5.56 | 0.048 | 0.24 | 59.001 | 32.835 | 3.788 | 2.665 | 1.711 |
| | 6.07 | 0.048 | 0.24 | 62.774 | 29.622 | 3.529 | 2.519 | 1.556 |
| | 6.59 | 0.048 | 0.24 | 52.538 | 36.517 | 5.252 | 3.553 | 2.141 |
| | | | | | | | | |
| **21.5 hr** | 3.94 | 0.048 | 0.24 | 15.796 | 61.140 | 10.614 | 7.222 | 5.228 |
| | 4.60 | 0.048 | 0.24 | 16.477 | 61.432 | 10.256 | 6.905 | 4.931 |
| | 5.10 | 0.048 | 0.24 | 17.630 | 60.956 | 10.028 | 6.623 | 4.763 |
| | 5.56 | 0.048 | 0.24 | 29.384 | 53.712 | 8.177 | 5.059 | 3.668 |
| | 6.07 | 0.048 | 0.24 | 34.823 | 51.570 | 6.383 | 4.269 | 2.956 |
| | 6.59 | 0.048 | 0.24 | 37.567 | 50.486 | 5.560 | 3.858 | 0.794 |
| | | | | | | | | |
| **94.5 hr** | 3.94 | 0.048 | 0.24 | 12.356 | 48.178 | 16.213 | 11.510 | 11.744 |
| | 4.60 | 0.048 | 0.24 | 12.008 | 48.170 | 16.311 | 12.020 | 11.491 |
| | 5.10 | 0.048 | 0.24 | 11.727 | 48.074 | 16.400 | 12.196 | 11.604 |
| | 5.56 | 0.048 | 0.24 | 13.095 | 48.057 | 16.023 | 11.899 | 10.925 |
| | 6.07 | 0.048 | 0.24 | 21.674 | 46.633 | 13.904 | 9.488 | 8.302 |
| | 6.59 | 0.048 | 0.24 | 30.227 | 46.796 | 10.286 | 7.134 | 5.556 |

### Example 6: Effect of pre-incubation with pullulanase on the production of maltotetraose with SAS3.

Maltodextrin was dissolved in tap water to reach a 32% DS value. The pH was adjusted to approximately 5.5 with 0.1 M sodium carbonate solution. A slurry of 4 g was added to a 18 × 150 mm glass test tube. Test tubes were inoculated as indicated in Table 2.

**Table 2.**

| **Exp** | **SAS3 Kq/MTDS** | **Pul Kg/MTDS** | **SAS3 (µl)** | **Pul (µl)** | **Water (µl)** | **Notes** |
|---|---|---|---|---|---|---|
| 1 | 0.024 | 0.24 | 10.2 | 20.5 | 29 | No incubation |
| 2 | 0.024 | 0.48 | 10.2 | 41 | 9 | No incubation |
| 3 | 0.024 | 0.24 | 10.2 | 20.5 | 29 | 2 hr with Pul |
| 4 | 0.024 | 0.24 | 10.2 | 20.5 | 29 | SAS3 and Pul added after 2 hr at 60□C |
| 5 | 0.024 | 0.24 | 10.2 | 20.5 | 29 | 2 hr with Pul |
| 6 | 0.024 | 0.24 | 10.2 | 20.5 | 29 | SAS3 and Pul added after 2 hr at 60□C |
| 7 | 0.024 | 0.48 | 10.2 | 41 | 9 | 2 hr with Pul |
| 8 | 0.024 | 0.48 | 10.2 | 41 | 9 | SAS3 and Pul added after 2 hr at 60□C |

In brief, tubes 1 and 2 were not pre-incubated. Tubes 3, 5, and 7 were pre-incubated for two hours after the addition of pullulanase and water, and time point sampling was initiated after the addition of SAS3. Tubes 4, 6, and 8 were pre-incubated for two hours with water only, and the time point sampling was initiated after the addition of both SAS3 and pullulanase. After adding the enzyme(s), tubes were stirred, capped with plastic covers, and placed in a water bath of 60°C. At various time points (*i.e*., 18.8, 44, and 72 hr), 0.02 mL sample was taken and resuspended in 1 mL 0.01 N sulfuric acid. The oligosaccharide contents were analyzed by HPLC as described in Example 2.

The data are compiled in Table 3. Overall, there was no significant difference on the amount of DPs for the pretreated sample as compared to the non-pretreated samples, *i.e*., between "2 hr H₂O Pre-Inc" and "2 hr Pul Pre-Inc" with the same amounts of enzymes. There was a slight increase in DP4 levels upon an increased dosage of the pullulanase. This result is most likely due to the increased dosage of pullulanase, because the pre-incubation with water also yielded a slight, yet similar, increase in DP4 production. Accordingly, pre-incubation with pullulanase alone does not improve the production of DP4. It appears necessary to have the presence of SAS3 to initiate the degradation of maltodextrins in order to allow the pullulanase to act on the partially hydrolyzed substrate.

**Table 3.**

| | **Exp** | **SAS3 Kg/MTDS** | **Pull Kg/MTDS** | **18.8 hr** | **44 hr** | **72 hr** |
|---|---|---|---|---|---|---|
| **DP5** | 2 hr H₂O Pre-Inc | 0.024 | 0.24 | 25.667 | 16.048 | 14.3185 |

| | **Exp** | **SAS3 Kq/MTDS** | **Pull Kg/MTDS** | **18.8 hr** | **44 hr** | **72 hr** |
|---|---|---|---|---|---|---|
| | 2 hr Pul Pre-Inc | 0.024 | 0.24 | 25.4725 | 15.957 | 14.376 |
| | 2 hr H₂O Pre-Inc | 0.024 | 0.48 | 23.869 | 15.162 | 14.266 |
| | 2 hr Pul Pre-Inc | 0.024 | 0.48 | 23.598 | 15.092 | 14.027 |
| | | | | | | |
| **DP4** | 2 hr H₂O Pre-Inc | 0.024 | 0.24 | 57.721 | 62.5675 | 60.9455 |
| | 2 hr Pul Pre-Inc | 0.024 | 0.24 | 57.831 | 62.691 | 60.9715 |
| | 2 hr H₂O Pre-Inc | 0.024 | 0.48 | 58.957 | 63.175 | 60.853 |
| | 2 hr Pul Pre-Inc | 0.024 | 0.48 | 59.177 | 63.269 | 61.245 |
| | | | | | | |
| **DP3** | 2 hr H₂O Pre-Inc | 0.024 | 0.24 | 7.906 | 9.9395 | 11.268 |
| | 2 hr Pul Pre-Inc | 0.024 | 0.24 | 7.909 | 9.921 | 11.2765 |
| | 2 hr H₂O Pre-Inc | 0.024 | 0.48 | 8.099 | 10.028 | 11.253 |
| | 2 hr Pul Pre-Inc | 0.024 | 0.48 | 8.115 | 10.016 | 11.248 |

### Example 7. Effect of higher temperatures on the production of maltotetraose by SAS3

Since SAS3 has been engineered to perform at higher temperatures, *e.g*., 75°C, it is expected that production of maltotetraose from maltodextrin would be more efficient at 75°C than at 60°C. To test this, maltodextrin was dissolved in tap water to reach a 32% DS value. The pH was adjusted to approximately 5.5 with 0.1 M sodium carbonate solution. A slurry of 4 g was then transferred to a 18 × 150 mm glass test tube. SAS3 was inoculated to the slurry at a dose of 0.007, 0.012, 0.024, or 0.048 Kg/MTDS. Test tubes were stirred, capped with plastic covers, and incubated in a water bath at 75°C. Approximately 20 µl samples were taken from each test tube at various time points (0, 1.3, 9.5, 22.5, 30, and 46.5 hours). The removed sample was suspended in 1 mL 0.01 N sulfuric acid in an Eppendorf tube. The suspension was centrifuged at 14,000 rpm for 2 minutes, and the supernatant was filtered through a 0.45 µm syringe filter. The filtrate was subject to HPLC analysis for oligosaccharide contents as described in Example 1.5.

The data are compiled in Table 4. In addition, the compiled data was plotted along with those obtained when maltotetraose production was performed at 60°C. *See* FIGS. 8A, 8B, and 8C. The effect of a higher hydrolysis rate due to temperature increase was observed for all three dosages of SAS3 tested. A slight elevation on percentage yield of DP4 occurred relatively early, *e.g*., 10-20 hours. At higher dosages (*i.e.* 0.024 and 0.048 Kg/MTDS) of SAS3, a decrease of DP4 production was observed, likely due to an increased rate of decomposition of DP4 into smaller DPs at 75°C. This effect has not been observed at lower temperatures. SAS3 appears to catalyze the decomposition at higher temperatures, as an increase in SAS3 dosage produced a more pronounced effect.

**Table 4.**

| **SAS Kq/MTDS** | **Time (hr)** | **DP5+** | **DP4** | **DP3** | **DP2** | **DP1** |
|---|---|---|---|---|---|---|
| **0.007** | 0 | 95.77 | 0 | 1.8 | 1.258 | 1.272 |
| | 1.3 | 86.187 | 8.695 | 2.304 | 1.703 | 1.111 |
| | 9.5 | 61.807 | 29.841 | 3.87 | 2.842 | 1.639 |
| | 22.5 | 49.216 | 39.894 | 4.976 | 3.745 | 2.169 |
| | 30 | 46.16 | 42.026 | 5.35 | 4.056 | 2.409 |
| | 46.5 | 43.509 | 43.657 | 5.769 | 4.427 | 2.638 |
| | | | | | | |
| **0.012** | 0 | 95.77 | 0 | 1.8 | 1.258 | 1.272 |
| | 1.3 | 79.536 | 14.692 | 2.646 | 1.929 | 1.196 |
| | 9.5 | 47.42 | 41.744 | 4.985 | 3.71 | 2.142 |
| | 22.5 | 38.595 | 47.285 | 6.327 | 4.851 | 2.941 |
| | 30 | 37.388 | 47.41 | 6.801 | 5.252 | 3.149 |
| | 46.5 | 36.586 | 46.728 | 7.341 | 5.807 | 3.538 |
| | | | | | | |
| **0.024** | 0 | 95.77 | 0 | 1.8 | 1.258 | 1.272 |
| | 1.3 | 71.191 | 22.175 | 3.111 | 2.241 | 1.283 |
| | 9.5 | 38.067 | 48.402 | 6.198 | 4.594 | 2.74 |
| | 22.5 | 34.207 | 47.947 | 7.845 | 6.179 | 3.821 |
| | 30 | 33.534 | 46.593 | 8.574 | 6.926 | 4.373 |
| | 46.5 | 32.723 | 44.796 | 9.503 | 7.814 | 5.163 |
| | | | | | | |
| **0.048** | 0 | 95.77 | 0 | 1.8 | 1.258 | 1.272 |
| | 1.3 | 59.156 | 32.56 | 3.906 | 2.74 | 1.637 |
| | 9.5 | 34.299 | 49.482 | 7.373 | 5.504 | 3.342 |
| | 22.5 | 31.168 | 44.049 | 9.973 | 8.446 | 6.363 |
| | 30 | 30.983 | 42.186 | 11.05 | 9.37 | 6.412 |
| | 46.5 | 30.203 | 38.739 | 12.265 | 10.803 | 7.99 |

### Example 8. Effect of pullulanase at higher temperatures in the formation of maltotetraose with SAS3.

The above example indicates that SAS3 is able to produce maltotetraose at an increased rate if the reaction is catalyzed at 75°C. The effect of the higher temperature on pullulanase was further studied. The reactions were performed similar as in Example 7, with various dosages of pullulanase added. The results are presented in FIGS. 9A, 9B, 9C, 9D, and 9E. Overall, the addition of a pullulanase did not result in a significant change on the production of maltotetraose at 75°C. Similar responses for production of DP4 and LDPs, as well as the hydrolysis of DP5+, were observed in the presence or absence of pullulanase. Each pair of curves overlaps well. This observation contrasts with what was observed at 60°C, as shown in FIG. 6. It is likely that the pullulanase is not a stable enzyme at 75°C and appears inactivated at this temperature.

**TABLE 5.**

| | | | | | | | | | |
|---|---|---|---|---|---|---|---|---|---|
| A3S | G70D | V1131 | G134C | G158T | A179N | G223P | W232P | G303L | R316P |
| A3T | G70K | N116D | R137C | G158F | A179R | G223I | W232Q | G303E | R316K |
| P7S | G70E | N119S | N138D | G158P | A179E | G223L | W232R | G303D | W323M |
| A8N | G70S | N119G | N138E | G158I | A179T | G223V | W232S | Q305E | T324L |
| G9A | G70Q | N119Y | N138S | G158A | R182S | G223C | W232Y | Q305T | T324M |
| H13R | G70A | N119E | C140R | G158V | R182H | G223T | W232T | Q305L | T324A |
| N26E | G70V | G121W | C140A | G158L | R182M | G223S | R233H | H307D | S325G |
| N26D | G70L | G121A | A141S | G158Q | R182D | G223Y | N234R | H307L | S334R |
| P32S | G70P | G121F | A141P | G158C | R182G | G223W | A236E | H307R | S334Q |
| N33Y | K71R | G121L | D142N | E160D | S183G | G223Q | S237G | H307K | S334H |
| D34N | K71M | G121T | D142G | S161V | G184Q | G223N | S237D | H307G | S334A |
| I38M | S72E | G121S | D142E | S161A | G188A | G223D | W238Q | H307P | S334M |
| I46F | S72K | G121E | P143T | S161T | G188H | G223H | W238G | H307I | S334L |
| D49V | S72N | G121K | G144E | S161K | G188T | G223K | W238K | H307S | S334P |
| D62N | S72T | G121R | N145D | S161P | G188S | G223R | W238R | H307M | H335M |
| F63L | G73M | G121H | N145S | S161G | F192Y | G223M | W238P | H307Q | W339E |
| F63A | G73S | G121M | Y146G | S161R | F192F | G223A | W238E | H307V | W339A |
| F63D | G73T | G121V | Y146E | S161H | F192M | G223E | Q239L | H307W | Y341E |
| F63E | G73N | G121P | Y146D | L163M | V195D | G223F | V253G | H307Y | Y341C |
| F63V | G73L | G121I | N148S | N164R | R196P | S225G | D255V | H307C | D343E |
| S64T | G73E | G121D | N148K | G166N | R196Q | S225E | A257V | H307F | R353T |
| S64N | G73D | Y122W | D149V | P168L | R196T | S225V | E260R | H307E | R358A |
| T67V | G74S | Y122A | D149L | Q169R | R196K | E226W | E260K | W308C | R358T |
| T67K | G75C | Y122Q | D149H | Q169K | R196Y | E226C | N264D | W308T | R358L |
| T67Q | G75S | Y122E | C150A | Q169V | R196S | E226D | V267I | W308K | R358V |
| T67H | G75R | P123S | D151W | Q169G | R196G | E226G | D269V | W308N | R358Q |
| T67R | G75Y | D124S | D151A | Q169E | R196A | Y227G | D269S | W308R | R358E |
| T67G | G75F | K125E | D151V | Q169N | R196V | Y227T | D269N | W308S | R358N |
| T67N | G75W | K125G | G153D | Q169D | Y198W | Y227D | K271L | W308G | R358G |
| D68C | G75E | K125A | S334K | 1170M | Y198F | Y227K | K271Q | W308Q | S367R |
| D68E | E76V | K125W | S334T | 1170E | A199P | Y227C | K271A | W308A | S367Q |
| G69M | G100A | K125D | G153A | 1170L | P200G | S229N | H272Q | A309T | S379G |
| G69I | G100S | K125Q | D154G | 1170K | P200A | S229P | G276R | A309E | D390E |
| G69H | G104R | K125P | D154E | 1170N | R202K | W232F | W282S | A309M | S399P |
| G69E | G104N | E126N | D154Y | L178N | S208T | W232G | V285A | A309V | S420G |
| G69A | G106K | E126D | F156Y | L178W | S213N | W232H | V290I | A309I | D422N |
| G69R | V107M | N128E | 1157L | L178Q | L220A | W2321 | T295C | A309P | D422Q |
| G69P | L110F | P130S | 1157V | L178F | L220T | W232K | Y297H | D312E | D422P |
| G69T | D112E | A131T | 1157M | A179P | K222Y | W232L | G300E | R316Q | G424S |
| G69K | | G134R | G158S | A179S | K222M | W232N | N302K | R316S | G424D |

| **TABLE 6.** | |
|---|---|
| **Backbone** | **Mutations** |
| **SAS3** | N33Y, D34N, G70D, G121F, G134R, A141P, Y146G, I157L, S161A, L178F, A179T, G223E, S229P, H307K, A309P, S334P |

### SEQUENCE LISTING

**SEQ ID NO: 1**
   Protein sequence of G4-amylase from *Pseudomonas saccharophila* (including signal sequence).
   >gi|77787|pir∥S05667 glucan 1, 4-alpha-maltotetraohydrolase (EC 3.2.1.60) precursor - *Pseudomonas saccharophila* 
MSHILRAAVLAAVLLPFPALADQAGKSPAGVRYHGGDEIILQGFHWNVVREAPNDWYNILRQQASTIA
ADGFSAIWMPVPWRDFSSWTDGGKSGGGEGYFWHDFNKNGRYGSDAQLRQAAGALGGAGVKVLYDVVP
NHMNRGYPDKEINLPAGQGFWRNDCADPGNYPNDCDDGDRFIGGESDLNTGHPQIYGMFRDELANLRS
GYGAGGFRFDFVRGYAPERVDSWMSDSADSSFCVGELWKGPSEYPSWDWRNTASWQQIIKDWSDRAKC
PVFDFALKERMQNGSVADWKHGLNGNPDPRWREVAVTFVDNHDTGYSPGQNGGQHHWALQDGLIRQAY
AYILTSPGTPVVYWSHMYDWGYGDFIRQLIQVRRTAGVRADSAISFHSGYSGLVATVSGSQQTLVVAL
NSDLANPGQVASGSFSEAVNASNGQVRVWRSGSGDGGNDGGEGGLVNVNFRCDNGVTQMGDSVYAVG
NVSQLGNWSPASAVRLTDTSSYPTWKGSIALPDGQNVEWKCLIRNEADATLVRQWQSGGNNQVQAAAG
ASTSGSF
**SEQ ID NO: 2**
   Synthetic sequence: PS4 with signal sequence and starch binding domain removed. 
DQAGKSPAGVRYHGGDEIILQGFHWNVVREAPNDWYNILRQQASTIAADGFSAIWMPVPWRDFSSWTD
GGKSGGGEGYFWHDFNKNGRYGSDAQLRQAAGALGGAGVKVLYDVVPNHMNRGYPDKEINLPAGQGFW
RNDCADPGNYPNDCDDGDRFIGGESDLNTGHPQIYGMFRDELANLRSGYGAGGFRFDFVRGYAPERVD
SWMSDSADSSFCVGELWKGPSEYPSWDWRNTASWQQIIKDWSDRAKCPVFDFALKERMQNGSVADWKH
GLNGNPDPRWREVAVTFVDNHDTGYSPGQNGGQHHWALQDGLIRQAYAYILTSPGTPVVYWSHMYDWG
YGDFIRQLIQVRRTAGVRADSAISFHSGYSGLVATVSGSQQTLVVALNSDLANPGQVASGSFSEAVNA
SNGQVRVWRSGSGDGGGNDGG
**SEQ ID NO: 3**
   Synthetic sequence: PS4 Variant 3 (SAS3) 
MDQAGKSPAGVRYHGGDEIILQGFHWNVVREAPYNWYNILRQQASTIAADGFSAIWMPVPWRDFSSWT
DGDKSGGGEGYFWHDFNKNGRYGSDAQLRQAAGALGGAGVKVLYDVVPNHMNRFYPDKEINLPAGQRF
WRNDCPDPGNGPNDCDDGDRFLGGEADLNTGHPQIYGMFRDEFTNLRSGYGAGGFRFDFVRGYAPERV
DSWMSDSADSSFCVGELWKEPSEYPPWDWRNTASWQQIIKDWSDRAKCPVFDFALKERMQNGSVADWK
HGLNGNPDPRWREVAVTFVDNHDTGYSPGQNGGQHKWPLQDGLIRQAYAYILTSPGTPVVYWPHMYDW
GYGDFIRQLIQVRRTAGVRADSAISFHSGYSGLVATVSGSQQTLVVALNSDLANPGQVASGSFSEAVN
ASNGQVRVWRSGSGDGGGNDGG
**SEQ ID NO: 4**
   DNA sequence of nucleotide encoding G4-amylase (PS4) from *Pseudomonas saccharophila;* GenBank Acc. No. X16732. 
1 gatcggcgta ggtttcgcat tcgttgccca ggcgatattt cgccggtgcg ccagcagcct
61 ggaagcaggc ctggtcgccg ccgccggccg tggcgccgac gcccgaacgc agatagccgt
121 ggaaatcgac cgccaggccc gggccgccga ccagcagggc ggcaagcagg caggcgggtt
181 ttaggacgaa cagggggtgc gcggtgtgct tcatgacgag gtccttgttt ttcttgttaa
241 tgccgaatcg atcacgcctt cgctgcgtgt cgcagggcgc agctcggtgg cgaaagcctc
301 ggggatggct ccgctggcgg catcctcccg accagagatt tcgctggcgc agctcgaggg
361 cgtaatcagg atgagtgcgg cgtaatccct ggggtggggc tacgcccggc agggcgcaga
421 tgattgccag gggccttcgg cctggccact acgccgcctg caactggcgg ggggaggttg
481 gtggtcgggg cgtgcagggg cagcctgcgg gtgccggtcg aagacccggc cggcgttcat
541 cctcgtccgg cggccttgcc gtaggatacc cgaacaagca caagaaccgg agtattgcga
601 tgagccacat cctgcgtgcc gccgtattgg cggcggtcct gctgccgttt cccgcactgg
661 ccgatcaggc cggcaagagc ccggccgggg tgcgctacca cggcggcgac gaaatcatcc
721 tccagggctt ccactggaac gtcgtccgcg aagcgcccaa cgactggtac aacatcctcc
781 gccaacaggc ctcgacgatc gcggccgacg gcttctcggc aatctggatg ccggtgccct
841 ggcgtgactt ctccagctgg accgacggcg gcaagtccgg cggcggcgaa ggctacttct
901 ggcacgactt caacaagaac ggccgctacg gcagcgacgc ccagctgcgc caggccgccg
961 gcgcactcgg tggcgccggg gtgaaggtgc tctacgatgt ggtgcccaat cacatgaacc
1021 gcggctaccc ggacaaggag atcaacctgc cggccggcca gggcttctgg cgcaacgact
1081 gcgccgaccc gggcaactac cccaacgact gcgacgacgg tgaccgcttc atcggcggcg
1141 agtcggacct gaacaccggc catccgcaga tttacggcat gtttcgcgac gagcttgcca
1201 acctgcgcag cggctacggc gccggcggct tccgcttcga cttcgttcgc ggctatgcgc
1261 ccgagcgggt cgacagctgg atgagcgaca gcgccgacag cagcttctgc gttggcgagc
1321 tgtggaaagg cccttctgaa tatccgagct gggactggcg caacacggcg agctggcagc
1381 agatcatcaa ggactggtcc gaccggccaa agtgcccggt gttcgacttc gctctcaagg
1441 agcgcatgca gaacggctcg gtcgccgact ggaagcatgg cctcaatggc aaccccgacc
1501 cgcgctggcg cgaggtggcg gtgaccttcg tcgacaacca cgacaccggc tattcgcccg
1561 ggcagaacgg cggccagcac cactgggcgc tgcaggacgg gctgatccgc caggcctacg
1621 cctacatcct caccagcccg ggcacgccgg tggtgtactg gtcgcacatg tacgactggg
1681 gctacggcga cttcatccgc cagctgatcc aggtgcggcg caccgccggc gtgcgcgccg
1741 attcggcgat cagcttccat agcggctaca gcggtctggt cgctaccgtc agcggcagcc
1801 agcagaccct ggtggtggcg ctcaactccg atctggccaa ccccggccag gttgccagcg
1861 gcagcttcag cgaggcggtc aacgccagca acggccaggt gcgcgtctgg cgcagcggta
1921 gcggcgatgg cggcgggaat gacggcggcg agggtggctt ggtcaatgtg aactttcgct
1981 gcgacaacgg cgtgacgcag atgggcgaca gcgtctacgc ggtgggcaac gtcagccagc
2041 tcggcaactg gagcccggcc tccgcggtac ggctgaccga caccagcagc tatccgacct
2101 ggaagggcag catcgccctg cctgacggtc agaacgtgga atggaagtgc ctgatccgca
2161 acgaggcgga cgcgacgctg gtgcgtcagt ggcaatcggg cggcaacaac caggtccagg
2221 ccgccgccgg cgcgagcacc agcggctcgt tctgacgaca tgcccgcccg gcctcggcta
2281 cgcctacgcc gggcggctcc tcccgaccca gggtgggcag ggaggaggcc ggcgacggc
2341 cgggccgccg atgctggcac gacaaccata aaagccttcg cgctgcgctg tcgtatcagg
2401 agctgttcat gttggcccag acccgctcga cccctttccg gcttggcttc ctggcccggc
2461 tgtacctgct gatcgccgca ctggtggcct tgctgatgct ggtagccggc accagcctgg
2521 ttgccatcgg ccgcctgcaa ggcaatgccg agcaaatctc gtcgaccgcg tcgcgtctgc
2581 tggtcagcga gagcttcttc ggtacgttgc agagcctgac gcagaacctg tccgacgccc
2641 tggccgagga ccggcctgac cagctcgacg gctatgtcgg ccggcatcgc acgctgcagg
2701 accaggccct cgagctgttc gcccagctgg agcgggtgac gccggcacat gccgagacca
2761 agcaagcctg gcggcgctgt tgccggagct cgaccgccgc agcctggcgc tgatcgatgc
2821 gcacgcgacc tgctcgcgcg tggggcgcaa cgccgtcgcc tgcgcgatct gcagctgcag
2881 ttctcgcggc tcaagcagga cctgctgcag gcgcagttcg tgacgggcga cgagctggtc
2941 gcctattcca tcaagcagtt catcatcccg ctcgagcagg tcgagcgctg ctgttcgatg
3001 ccatcggcgt gtcttcgatc aaggcactcg atgaagcggg tgcgcagatc
**SEQ ID NO: 5**
   nucleotide sequence of *Bacillus deramificans* pullulanase gene (coding region for the mature protein, w/o signal peptide) (SEQ ID NO: 10 of U.S. Patent No. 5,736,375) 
GATGGGAACACGACAACGATCATTGTCCACTATTTTGCCCTGCTGGTGATTATCAACCTTG
GAGTCTATGGATGTGGCCAAAAGACGGAGGTGGGGCTGAATACGATTTCAATCAACCGGCTG
ACTCTTTTGGAGCTGTTGCAAGTGCTGATATTCCAGGAAACCCAAGTCAGGTAGGAATTATC
GTTCGCACTCAAGATTGGACCAAAGATGTGAGCGCTGACCGCTACATAGATTTAAGCAAAGG
AAATGAGGTGTGGCTTGTAGAAGGAAACAGCCAAATTTTTATAATGAAAAAGATGCTGAGG
ATGCAGCTAAACCCGCTGTAAGCAACGCTTATTTAGATGCTTCAAACCAGGTGCTGGTTAAA
CTTAGCCAGCCGTTAACTCTTGGGGAAGGNNNAAGCGGCTTTACGGTTCATGACGACACAGC
AAATAAGGATATTCCAGTGACATCTGTGAAGGATGCAAGTCTTGGTCAAGATGTAACCGCTG
TTTTGGCAGGTACCTTCCAACATATTTTTGGAGGTTCCGATTGGGCACCTGATAATCACAGT
ACTTTATTAAAAAAGGTGACTAACAATCTCTATCAATTCTCAGGAGATCTTCCTGAAGGAAA
CTACCAATATAAAGTGGCTTTAAATGATAGCTGGAATAATCCGAGTTACCCATCTGACAACA
TTAATTTAACAGTCCCTGCCGGCGGTGCACACGTCACTTTTTCGTATATTCCGTCCACTCAT
GCAGTCTATGACACAATTAATAATCCTAATGCGGATTTACAAGTAGAAAGCGGGGTTAAAAC
GGATCTCGTGACGGTTACTCTAGGGGAAGATCCAGATGTGAGCCATACTCTGTCCATTCAAA
CAGATGGCTATCAGGCAAAGCAGGTGATACCTCGTAATGTGCTTAATTCATCACAGTACTAC
TATTCAGGAGATGATCTTGGGAATACCTATACACAGAAAGCAACAACCTTTAAAGTCTGGGC
ACCAACTTCTACTCAAGTAAATGTTCTTCTTTATGACAGTGCAACGGGTTCTGTAACAAAAA
TCGTACCTATGACGGCATCGGGCCATGGTGTGTGGGAAGCAACGGTTAATCAAAACCTTGAA
AATTGGTATTACATGTATGAGGTAACAGGCCAAGGCTCTACCCGAACGGCTGTTGATCCTTA
TGCAACTGCGATTGCACCAAATGGAACGAGAGGCATGATTGTGGACCTGGCTAAAACAGATC
CTGCTGGCTGGAACAGTGATAAACATATTACGCCAAAGAATATAGAAGATGAGGTCATCTAT
GAAATGGATGTCCGTGACTTTTCCATTGACCCTAATTCGGGTATGAAAAATAAAGGGAAGTA
TTTGGCTCTTACAGAAAAAGGAACAAAGGGCCCTGACAACGTAAAGACGGGGATAGATTCCT
TAAAACAACTTGGGATTACTCATGTTCAGCTTATGCCTGTTTTCGCATCTAACAGTGTCGAT
GAAACTGATCCAACCCAAGATAATTGGGGTTATGACCCTCGCAACTATGATGTTCCTGAAGG
GCAGTATGCTACAAATGCGAATGGTAATGCTCGTATAAAAGAGTTTAAGGAAATGGTTCTTT
CACTCCATCGTGAACACATTGGGGTTAACATGGATGTTGTCTATAATCATACCTTTGCCACG
CAAATCTCTGACTTCGATAAAATTGTACCAGAATATTATTACCGTACGATGATGCAGGTAAT
TATACCAACGGATCAGGTACTGGAAATGAAATTGCANGCNGAAAGGCCAATGGTTCAAAAAT
TTATTATTGATTCCCTTAAGTATTGGGTCAATGAGTATCATATTGACGGCTTCCGTTTTGAC
TTAATGGCGCTGCTTGGAAAAGACACGATGTCCAAAGCTGCCTCGGAGCTTCATGCTATTAA
TCCAGGAATTGCACTTTACGGTGAGCCATGGACGGGTGGAACCTCTGCACTGCCAGATGATC
AGCTTCTGACAAAAGGAGCTCAAAAAGGCATGGGAGTAGCGGTGTTTAATGACAATTTACGA
AACGCGTTGGACGGCAATGTCTTTGATTCTTCCGCTCAAGGTTTTGCGACAGGTGCAACAGG
CTTAACTGATGCAATTAAGAATGGCGTTGAGGGGAGTATTAATGACTTTACCTCTTCACCAG
GTGAGACAATTAACTATGTCACAAGTCATGATAACTACACCCTTTGGGACAAAATAGCCCTA
AGCAATCCTAATGATTCCGAAGCGGATCGGATTAAAATGGATGAACTCGCACAAGCAGTTGT
TATGACCTCACAAGGCGTTCCATTCATGCAAGGCGGGGAAGAAATGCTTCGTANAAAAGGCG
GCAACGACAATAGTTATAATGCAGGCGATGCGGTCAATGAGTTTGATTGGAGCAGGAAAGCT
CAATATCCAGATGTTTTCAACTATTATAGCGGGCTAATCCACCTTCGTCTTGATCACCCAGC
CTTCCGCATGACGACAGCTAATGAAATCAATAGCCACCTCCAATTCCTAAATAGTCCAGAGA
ACACAGTGGCCTATGAATTAACTGATCATGTTAATAAAGACAAATGGGGAAATATCATTGTT
GTTTATAACCCAAATAAAACTGTAGCAACCATCAATTTGCCGAGCGGGAAATGGGCAATCAA
 
TGCTACGAGCGGTAAGGTAGGAGAATCCACCCTTGGTCAAGCAGAGGGAAGTGTCCAAGTAC
CAGGTATATCTATGATGATCCTTCATCAAGAGGTAAGCCCAGACCACGGTAAAAAG
**SEQ ID NO: 6**
   amino acid sequence of *Bacillus deramificans* pullulanase, mature protein, w/o signal peptide (SEQ ID NO: 11 of U.S. Patent No. 5,736,375) 
DGNTTTIIVHYFCPAGDYQPWSLWMWPKDGGGAEYDFNQPADSFGAVASADIPGNPSQVGII
VRTQDWTKDVSADRYIDLSKGNEVWLVEGNSQIFYNEKDAEDAAKPAVSNAYLDASNQVLVK
LSQPLTLGEGXSGFTVHDDTANKDIPVTSVKDASLGQDVTAVLAGTFQHIFGGSDWAPDNHS
TLLKKVTNNLYQFSGDLPEGNYQYKVALNDSWNNPSYPSDNINLTVPAGGAHVTFSYIPSTH
AVYDTINNPNADLQVESGVKTDLVTVTLGEDPDVSHTLSIQTDGYQAKQVIPRNVLNSSQYY
YSGDDLGNTYTQKATTFKVWAPTSTQVNVLLYDSATGSVTKIVPMTASGHGVWEATVNQNLE
NWYYMYEVTGQGSTRTAVDPYATAIAPNGTRGMIVDLAKTDPAGWNSDKHITPKNIEDEVIY
EMDVRDFSIDPNSGMKNKGKYLALTEKGTKGPDNVKTGIDSLKQLGITHVQLMPVFASNSVD
ETDPTQDNWGYDPRNYDVPEGQYATNANGNARIKEFKEMVLSLHREHIGVNMDVVYNHTFAT
QISDFDKIVPEYYYRTMMQVIIPTDQVLEMKLXAERPMVQKFIIDSLKYWVNEYHIDGFRFD
LMALLGKDTMSKAASELHAINPGIALYGEPWTGGTSALPDDQLLTKGAQKGMGVAVFNDNLR
NALDGNVFDSSAQGFATGATGLTDAIKNGVEGSINDFTSSPGETINYVTSHDNYTLWDKIAL
SNPNDSEADRIKMDELAQAVVMTSQGVPFMQGGEEMLRXKGGNDNSYNAGDAVNEFDWSRKA
QYPDVFNYYSGLIHLRLDHPAFRMTTANEINSHLQFLNSPENTVAYELTDHVNKDKWGNIIV
VYNPNKTVATINLPSGKWAINATSGKVGESTLGQAEGSVQVPGISMMILHQEVSPDHGKK
**SEQ ID NO: 7**
   full length nucleotide sequence of *Bacillus deramificans* (1163-4033) (AR098240)(SEQ ID NO: 9 of U.S. Patent No. 6,074,854) 
GGATCCTGTTAGACTATTTGAGGAGTTGCAACACTTGATGTTTATCCAAAGGAAGGGCCG
GAGATCATCGCTGGTCGAGGTGCTTTCGGTGAAGCATTTTCGCTATTTTGGGTATAACCGGG
CGCATTACGATCAATTGTTTGAAGAGCATCTTGATTTACTTCAAAAGCTGAATGCTTCGAAA
AGAATAACATGGAGCGGGCTTTATCGAACACCTATACATGATGCAGATATCGCACCCCGCCC
TGTTCAGAAAAACATTCCTTTGTGGGTTGGGGTGGGTGGGACNMNTGAAASCNSYKCKYYGT
GCRNVSNNNTATGGTGCCGGCTTAGCATGGGTATTTTGTCAGGCGATTGGCTTCGGTTTAAG
GCACTTTCGGACCTTTATCGGCAGGCCGGCCAACAAGCANGGTATTCACCGAACGATCTGAA
AGTAGGAGTGACAGGGCATGCGTTTATTGGAAAGACGTCGCAGCAGGCACTCAATGACTATT
ACCCCTATCACGCGAATTATTGGCTAACACTGAACCAACAATTAGGGCAGCCGTTACCCCAG
CAATACGTGAGGGAATTTAATTTATTAGCCTCCCCAGAGCAAGCCTTATATGTGGGAAGCTC
TCAACAAGTGGGCAGGNAAAAATTTTGCGCCAACATGAGGNATTTGGTNATAAACGTTTTAT
CGCACAGATCGACATTGGCGGAATGCCCTTTAAAACAGTGGCCAAGAATATTGAGCGGTTAG
GCCACTGAGGTTGCACCTGTCGTACGAAGAGCAACAAGAGGGTAATGGTAATAATCTATTTA
ACTGTTTATTAGAAAACTTGGTATCTGTTTAATTAAATAACAGGAGCCTGGAAGTGGGCCAA
GGCTCCTTTCTAGGGAAACCTTTTTCTATTTATATAGGCGTTGTTGCCTAAGGCTAAAGTAG
GATTTTATTAAAAATATAGGAATTGCTCTTTTATTCGACACAATTATTCAATGGAATACGAT
AAAATGGAGAGTGTATGTAAGCGTTATATTTTATTGGGGGGCTGATAGAAGAAAAGGGATGC
GACAGGGTCTATTAGCTAGTTTGGTATTCGATTTCAGATCAATGCAACGTACGAGTTTTTTA
TTGACTGCTTTGTGCAAGCGATTGCATTGAAACAAAGGAGGACATTATGGCTAAAAAACTAA
TTTATGTGTGTTTAAGTGTTTGTTTAGTGTTGACCTGGGCTTTTAATGTAAAAGGGCAATCT
GCTCATGCTGATGGGAACACGACAACGATCATTGTCCACTATTTTTGCCCTGCTGGTGATTA
TCAACCTTGGAGTCTATGGATGTGGCCAAAAGACGGAGGTGGGGCTGAATACGATTTCAATC
AACCGGCTGACTCTTTTGGAGCTGTTGCAAGTGCTGATATTCCAGGAAACCCAAGTCAGGTA
GGAATTATCGTTCGCACTCAAGATTGGACCAAAGATGTGAGCGCTGACCGCTACATAGATTT
AAGCAAAGGAAATGAGGTGTGGCTTGTAGAAGGAAACAGCCAAATTTTTTATAATGAAAAAG
ATGCTGAGGATGCAGCTAAACCCGCTGTAAGCAACGCTTATTTAGATGCTTCAAACCAGGTG
CTGGTTAAACTTAGCCAGCCGTTAACTCTTGGGGAAGGNNNAAGCGGCTTTACGGTTCATGA
CGACACAGCAAATAAGGATATTCCAGTGACATCTGTGAAGGATGCAAGTCTTGGTCAAGATG
TAACCGCTGTTTTGGCAGGTACCTTCCAACATATTTTTGGAGGTTCCGATTGGGCACCTGAT
AATCACAGTACTTTATTAAAAAAGGTGACTAACAATCTCTATCAATTCTCAGGAGATCTTCC
TGAAGGAAACTACCAATATAAAGTGGCTTTAAATGATAGCTGGAATAATCCGAGTTACCCAT
CTGACAACATTAATTTAACAGTCCCTGCCGGCGGTGCACACGTCACTTTTTCGTATATTCCG
TCCACTCATGCAGTCTATGACACAATTAATAATCCTAATGCGGATTTACAAGTAGAAAGCGG
GGTTAAAACGGATCTCGTGACGGTTACTCTAGGGGAAGATCCAGATGTGAGCCATACTCTGT
CCATTCAAACAGATGGCTATCAGGCAAAGCAGGTGATACCTCGTAATGTGCTTAATTCATCA
CAGTACTACTATTCAGGAGATGATCTTGGGAATACCTATACACAGAAAGCAACAACCTTTAA
AGTCTGGGCACCAACTTCTACTCAAGTAAATGTTCTTCTTTATGACAGTGCAACGGGTTCTG
TAACAAAAATCGTACCTATGACGGCATCGGGCCATGGTGTGTGGGAAGCAACGGTTAATCAA
AACCTTGAAAATTGGTATTACATGTATGAGGTAACAGGCCAAGGCTCTACCCGAACGGCTGT
TGATCCTTATGCAACTGCGATTGCACCAAATGGAACGAGAGGCATGATTGTGGACCTGGCTA
AAACAGATCCTGCTGGCTGGAACAGTGATAAACATATTACGCCAAAGAATATAGAAGATGAG
GTCATCTATGAAATGGATGTCCGTGACTTTTCCATTGACCCTAATTCGGGTATGAAAAATAA
AGGGAAGTATTTGGCTCTTACAGAAAAAGGAACAAAGGGCCCTGACAACGTAAAGACGGGGA
TAGATTCCTTAAAACAACTTGGGATTACTCATGTTCAGCTTATGCCTGTTTTCGCATCTAAC
 
AGTGTCGATGAAACTGATCCAACCCAAGATAATTGGGGTTATGACCCTCGCAACTATGATGT
TCCTGAAGGGCAGTATGCTACAAATGCGAATGGTAATGCTCGTATAAAAGAGTTTAAGGAAA
TGGTTCTTTCACTCCATCGTGAACACATTGGGGTTAACATGGATGTTGTCTATAATCATACC
TTTGCCACGCAAATCTCTGACTTCGATAAAATTGTACCAGAATATTATTACCGTACGATGAT
GCAGGTAATTATACCAACGGATCAGGTACTGGAAATGAAATTGCANGCNGAAAGGCCAATGG
TTCAAAAATTTATTATTGATTCCCTTAAGTATTGGGTCAATGAGTATCATATTGACGGCTTC
CGTTTTGACTTAATGGCGCTGCTTGGAAAAGACACGATGTCCAAAGCTGCCTCGGAGCTTCA
TGCTATTAATCCAGGAATTGCACTTTACGGTGAGCCATGGACGGGTGGAACCTCTGCACTGC
CAGATGATCAGCTTCTGACAAAAGGAGCTCAAAAAGGCATGGGAGTAGCGGTGTTTAATGAC
AATTTACGAAACGCGTTGGACGGCAATGTCTTTGATTCTTCCGCTCAAGGTTTTGCGACAGG
TGCAACAGGCTTAACTGATGCAATTAAGAATGGCGTTGAGGGGAGTATTAATGACTTTACCT
CTTCACCAGGTGAGACAATTAACTATGTCACAAGTCATGATAACTACACCCTTTGGGACAAA
ATAGCCCTAAGCAATCCTAATGATTCCGAAGCGGATCGGATTAAAATGGATGAACTCGCACA
AGCAGTTGTTATGACCTCACAAGGCGTTCCATTCATGCAAGGCGGGGAAGAAATGCTTCGTA
NAAAAGGCGGCAACGACAATAGTTATAATGCAGGCGATGCGGTCAATGAGTTTGATTGGAGC
AGGAAAGCTCAATATCCAGATGTTTTCAACTATTATAGCGGGCTAATCCACCTTCGTCTTGA
TCACCCAGCCTTCCGCATGACGACAGCTAATGAAATCAATAGCCACCTCCAATTCCTAAATA
GTCCAGAGAACACAGTGGCCTATGAATTAACTGATCATGTTAATAAAGACAAATGGGGAAAT
ATCATTGTTGTTTATAACCCAAATAAAACTGTAGCAACCATCAATTTGCCGAGCGGGAAATG
GGCAATCAATGCTACGAGCGGTAAGGTAGGAGAATCCACCCTTGGTCAAGCAGAGGGAAGTG
TCCAAGTACCAGGTATATCTATGATGATCCTTCATCAAGAGGTAAGCCCAGACCACGGTAAA
AAGTAATAGAAAAAGTAAAATCCCCTCAAGATGTTTGAGGGGGATTTAGTTACTTATTATC
CAATTAATTTGCGGCTTCGGTGTTTTCAATGGGCTCCGTATCCGTTCGGTTGTGTGATCGGA
CAAATGGGAGTGAATAGGTCACAAGAGCAGCAGCCATTTCAAGCAGACCAGCGAAAGTAAAC
ATTCGTTCTGGTGCAAATCGGGTCATCAACCAACCGGTAATTGCTTGGGAAATAGGGATGGA
CCCTGACATCACGATAATCATAATACTAATAACACGACCGAATAACTTAGGTGGAATAAGCG
TATGGTTAACGCTTGGAGCAATAATATTAACCGCCGTTTCATGAGCGCCAACAAGCACTAGA
AGGGCTAAAATAACCCATAAGTTGTGTGTAAATCCTATAAAAAATAACATAAGGCCCTGCAG
**SEQ ID NO: 8**
   amino acid sequence of *Bacillus deramificans* pullulanase, signal peptide, 29 aa (U.S. Patent No. 6,074,854)
   MAKKLIYVCLSVCLVLTWAFNVKGQSAHA

## Claims

1. A method of processing a starch comprising saccharifying a starch liquefact or a maltodextrin by contacting a *Pseudomonas saccharophila* amylase (PS4) variant and a pullulanase to the starch liquefact or the maltodextrin to form a saccharide syrup, wherein the PS4 variant has at least 80% amino acid sequence identity to a naturally occurring PS4 having an amino acid sequence of SEQ ID NO: 2 and has amino acid deletions, additions, insertions, or substitutions compared to the amino acid sequence of SEQ ID NO: 2, or comprises up to 25 amino acid deletions, additions, insertions, or substitutions compared to the amino acid sequence of SEQ ID NO: 2, and wherein the pullulanase comprises an amino acid sequence having at least about 80% amino acid sequence identity to SEQ ID NO: 6, wherein the saccharide syrup comprises at least about 54% by weight maltotetraose based on total saccharide content.

2. The method of claim 1, wherein the PS4 variant comprises a G223E amino acid substitution compared to the PS4 having an amino acid sequence of SEQ ID NO: 2.

3. The method of claim 2, wherein the PS4 variant comprises an amino acid sequence having up to 15 additional amino acid substitutions compared to the PS4 having an amino acid sequence of SEQ ID NO: 2.

4. The method of claim 3, wherein the PS4 variant comprises one or more of the following amino acid substitutions: N33Y, D34N, G70D, G121F, G134R, A141P, Y146G, I157L, S161A, L178F, A179T, S229P, H307K, A309P, and/or S334P compared to the PS4 having an amino acid sequence of SEQ ID NO: 2.

5. The method of claim 4, wherein the PS4 variant comprises an amino acid sequence of SEQ ID NO: 3.

6. The method of claim 1, wherein the PS4 variant exhibits altered properties compared to the PS4 having an amino acid sequence of SEQ ID NO: 2.

7. The method of claim 6, wherein the altered properties include improved thermostability and/or improved stability at a pH of 5.0 to 7.0 compared to the PS4 having an amino acid sequence of SEQ ID NO: 2.

8. The method of claim 6, wherein the altered properties include an increased exo-alpha-amylase activity or a decreased endo-alpha-amylase activity compared to the PS4 having an amino acid sequence of SEQ ID NO: 2.

9. The method of claim 1, wherein the pullulanase is a naturally occurring enzyme from *Bacillus,* and optionally from *Bacillus deramificans.*

10. The method of claim 1, wherein:
(a) the pullulanase comprises an amino acid sequence of SEQ ID NO: 6; or
(b) the pullulanase consists of an amino acid sequence of SEQ ID NO: 6; or
(c) the pullulanase is a variant enzyme, optionally a variant of a *Bacillus deramificans* pullulanase having an amino acid sequence of SEQ ID NO: 6 and having amino acid deletions, additions, insertions, or substitutions compared to the amino acid sequence of SEQ ID NO: 6.

11. The method of claim 10, wherein the variant pullulanase exhibits altered properties compared to a pullulanase having an amino acid sequence of SEQ ID NO: 6.

12. The method claim 11, wherein the altered properties include improved thermostability, pH dependent activity, specific activity, substrate specificity, or any combination thereof, compared to a pullulanase having an amino acid sequence of SEQ ID NO: 6.

13. The method of claim 1, wherein the pullulanase is expressed and/or isolated from a host cell of *Bacillus,* and optionally from the host cell of *Bacillus licheniformis.*

14. The method of claim 13, wherein a Carlsberg protease gene and/or a Glu C protease gene of the host cell of *Bacillus licheniformis* has been altered to eliminate protease activity

15. The method of claim 1, wherein the PS4 variant and/or the pullulanase are purified.

16. The method of claim 1, wherein the PS4 variant is added to the starch liquefact in a range from 0.05 to 1 Kg/Metric Tons Dry Solids.

17. The method of claim 1, wherein the pullulanase, measured as Kg/Metric Tons Dry Solids, is added to the starch liquefact at an amount in a range from 5 to about 50 times of the PS4 variant.

18. The method of claim 1, wherein:
(a) the starch liquefact is saccharified at 60°C to 75°C; and/or
(b) the starch liquefact is saccharified at pH 3.9 to pH 5.5.

19. The method of claim 1, wherein the saccharide syrup contains at least 20% more maltotetraose than a saccharide syrup obtained with the PS4 variant but without the pullulanase.

20. The method of claim 1 further comprising contacting an isoamylase, a protease, a cellulase, a hemicellulase, a lipase, a cutinase, or any combination thereof, to the starch liquefact or the maltodextrin.

21. The method of claim 1, wherein the starch is from corn, wheat, barley, rye, milo, sago, cassava, tapioca, sorghum, rice, peas, bean, banana, or potatoes.

## Patentansprüche

1. Verfahren zur Verarbeitung einer Stärke, umfassend Verzuckern eines Stärkeverflüssigungsansatzes oder eines Maltodextrins durch In-Kontakt-Bringen einer *Pseudomonas saccharophila-*Amylase(PS4)-Variante und einer Pullulanase mit dem Stärkeverflüssigungsansatz bzw. dem Maltodextrin, so dass ein Saccharidsirup gebildet wird, wobei die PS4-Variante eine Aminosäuresequenzidentität von wenigstens 80 % mit einer natürlich vorkommenden PS4 mit einer Aminosäuresequenz unter SEQ ID NO: 2 besitzt und gegenüber der Aminosäuresequenz unter SEQ ID NO: 2 Aminosäuredeletionen, -additionen, -Insertionen oder - substitutionen aufweist oder bis zu 25 Aminosäuredeletionen, -additionen, -Insertionen oder - substitutionen im Vergleich zur Aminosäuresequenz unter SEQ ID NO: 2 umfasst und wobei die Pullulanase eine Aminosäuresequenz mit einer Aminosäuresequenzidentität von wenigstens etwa 80 % mit SEQ ID NO: 6 umfasst, wobei der Saccharidsirup wenigstens etwa 54 Gew.-% Maltotetraose bezogen auf den Gesamtsaccharidgehalt umfasst.

2. Verfahren nach Anspruch 1, wobei die PS4-Variante eine G223E Aminosäuresubstitution im Vergleich zur PS4 mit einer Aminosäuresequenz unter SEQ ID NO: 2 umfasst.

3. Verfahren nach Anspruch 1, wobei die PS4-Variante eine Aminosäuresequenz mit bis zu 15 zusätzlichen Aminosäuresubstitutionen im Vergleich zur PS4 mit einer Aminosäuresequenz unter SEQ ID NO: 2 umfasst

4. Verfahren nach Anspruch 3, wobei die PS4-Variante eine oder mehrere der folgenden Aminosäuresubstitutionen im Vergleich zur PS4 mit einer Aminosäuresequenz unter SEQ ID NO: 2 umfasst: N33Y, D34N, G70D, G121F, G134R, A141P, Y146G, I157L, S161A, L178F, A179T, S229P, H307K, A309P und/oder S334P.

5. Verfahren nach Anspruch 4, wobei die PS4-Variante eine Aminosäuresequenz unter SEQ ID NO: 3 umfasst.

6. Verfahren nach Anspruch 1, wobei die PS4-Variante veränderte Eigenschaften im Vergleich zur PS4 mit einer Aminosäuresequenz unter SEQ ID NO: 2 zeigt.

7. Verfahren nach Anspruch 6, wobei zu den veränderten Eigenschaften eine verbesserte Thermostabilität und/oder verbesserte Stabilität bei einem pH von 5,0 bis 7,0 im Vergleich zur PS4 mit einer Aminosäuresequenz unter SEQ ID NO: 2 gehören/gehört.

8. Verfahren nach Anspruch 6, wobei zu den veränderten Eigenschaften eine erhöhte Exo-alpha-Amylase-Aktivität oder eine verminderte Endo-alpha-Amylase-Aktivität im Vergleich zur PS4 mit einer Aminosäuresequenz unter SEQ ID NO: 2 gehört.

9. Verfahren nach Anspruch 1, wobei es sich bei der Pullulanase um ein natürlich vorkommendes Enzym aus *Bacillus* und gegebenenfalls aus *Bacillus deramificans* handelt.

10. Verfahren nach Anspruch 1, wobei:
(a) die Pullulanase eine Aminosäuresequenz unter SEQ ID NO: 6 umfasst; oder
(b) die Pullulanase aus einer Aminosäuresequenz unter SEQ ID NO: 6 besteht; oder
es sich bei der Pullulanase um eine Enzymvariante, gegebenenfalls eine Variante einer *Bacillus deramificans-*Pullulanase mit einer Aminosäuresequenz unter SEQ ID NO: 6 und mit Aminosäuredeletionen, -additionen, -Insertionen oder -substitutionen im Vergleich zur Aminosäuresequenz unter SEQ ID NO: 6 handelt.

11. Verfahren nach Anspruch 10, wobei die Pullulanasevariante veränderte Eigenschaften im Vergleich zu einer Pullulanase mit einer Aminosäuresequenz unter SEQ ID NO: 6 zeigt.

12. Verfahren nach Anspruch 11, wobei zu den veränderten Eigenschaften eine verbesserte Thermostabilität, pHabhängige Aktivität, spezifische Aktivität, Substratspezifität oder irgendeine Kombination davon im Vergleich zu einer Pullulanase mit einer Aminosäuresequenz unter SEQ ID NO: 6 gehört.

13. Verfahren nach Anspruch 1, wobei die Pullulanase aus einer *Bacillus*-Wirtszelle und gegebenenfalls aus der *Bacillus licheniformis*-Wirtszelle exprimiert und/oder isoliert wird.

14. Verfahren nach Anspruch 13, wobei ein Carlsberg-Protease-Gen und/oder ein Glu-Protease-Gen der *Bacillus licheniformis*-Wirtszelle zum Ausschalten von Protease-Aktivität verändert wurde.

15. Verfahren nach Anspruch 1, wobei die PS4-Variante und/oder die Pullulanase gereinigt sind.

16. Verfahren nach Anspruch 1, wobei die PS4-Variante in einem Bereich von 0,05 bis 1 kg pro Tonne Trockensubstanz zum Stärkeverflüssigungsansatz gegeben wird.

17. Verfahren nach Anspruch 1, wobei die Pullulanase in einer Menge in einem Bereich von dem 5- bis etwa 50-Fachen der PS4-Variante, in kg pro Tonne Trockensubstanz gemessen, zum Stärkeverflüssigungsansatz gegeben wird.

18. Verfahren nach Anspruch 1, wobei:
(a) der Stärkeverflüssigungsansatz bei 60°C bis 75°C verzuckert wird; und/oder
(b) der Stärkeverflüssigungsansatz bei pH 3,9 bis pH 5,5 verzuckert wird.

19. Verfahren nach Anspruch 1, wobei der Saccharidsirup wenigstens 20 % mehr Maltotetraose als ein mit der PS4-Variante, aber ohne die Pullulanase erhaltener Saccharidsirup enthält.

20. Verfahren nach Anspruch 1, ferner umfassend In-Kontakt-Bringen einer Isoamylase, einer Protease, einer Cellulase, einer Hemicellulase, einer Lipase, einer Cutinase oder irgendeiner Kombination davon mit dem Stärkeverflüssigungsansatz oder dem Maltodextrin.

21. Verfahren nach Anspruch 1, wobei die Stärke aus Mais, Weizen, Gerste, Roggen, Hirse, Sago, Maniok, Tapioka, Sorghum, Reis, Erbsen, Bohne, Banane oder Kartoffeln stammt.

## Revendications

1. Procédé de traitement d'un amidon comprenant la saccharification d'un produit de liquéfaction d'amidon ou d'une maltodextrine par mise en contact d'un variant d'amylase (PS4) de *Pseudomonas saccharophila* et d'une pullulanase avec le produit de liquéfaction d'amidon ou de la maltodextrine pour former un sirop de saccharide, le variant de PS4 ayant au moins 80 % d'identité de séquence d'acides aminés avec une PS4 d'origine naturelle ayant une séquence d'acides aminés de la SEQ ID NO: 2 et ayant des délétions, des additions, des insertions ou des substitutions par comparaison avec la séquence d'acides aminés de la SEQ ID NO: 2, ou
comprenant jusqu'à 25 délétions, additions, insertions ou substitutions d'acides aminés par comparaison avec la séquence d'acides aminés de la SEQ ID NO: 2, et la pullulanase comprenant une séquence d'acides aminés ayant au moins 80 % d'identité de séquence d'acides aminés avec la SEQ ID NO: 6, le sirop de saccharide comprenant au moins 54 % en poids de maltotétraose sur la base d'une teneur totale en saccharide.

2. Procédé selon la revendication 1, le variant de PS4 comprenant une substitution d'acides aminés G223E par comparaison avec la PS4 ayant une séquence d'acides aminés de la SEQ ID NO: 2.

3. Procédé selon la revendication 2, le variant de PS4 comprenant une séquence d'acides aminés ayant jusqu'à 15 substitutions d'acides aminés supplémentaires par comparaison avec la PS4 ayant une séquence d'acides aminés de la SEQ ID NO: 2.

4. Procédé selon la revendication 3, le variant de PS4 comprenant l'une ou plusieurs des substitutions d'acides aminés suivantes : N33Y, D34N, G70D, G121F, G134R, A141P, Y146G, I157L, S161A, L178F, A179T, S229P, H307K, A309P et/ou S334P par comparaison avec la PS4 ayant une séquence d'acides aminés de la SEQ ID NO: 2.

5. Procédé selon la revendication 4, le variant de PS4 comprenant une séquence d'acides aminés de la SEQ ID NO: 3.

6. Procédé selon la revendication 1, le variant de PS4 présentant des propriétés modifiées par comparaison avec la PS4 ayant une séquence d'acides aminés de la SEQ ID NO: 2.

7. Procédé selon la revendication 6, les propriétés modifiées comprenant une stabilité thermique modifiée et/ou une stabilité modifiée à un pH de 5,0 à 7,0 par comparaison avec la PS4 ayant une séquence d'acides aminés de la SEQ ID NO: 2.

8. Procédé selon la revendication 6, les propriétés modifiées comprenant une activité d'exo-alpha-amylase augmentée ou une activité dans d'endo-alpha-amylase diminuée par comparaison avec la PS4 ayant une séquence d'acides aminés de la SEQ ID NO: 2.

9. Procédé selon la revendication 1, la pullulanase étant une enzyme d'origine naturelle provenant de *Bacillus,* et éventuellement de *Bacillus deramificans.*

10. Procédé selon la revendication 1,
(a) la pullulanase comprenant une séquence d'acides aminés de la SEQ ID NO: 6 ; ou
(b) la pullulanase étant constituée d'une séquence d'acides aminés de la SEQ ID NO: 6 ; ou
(c) la pullulanase étant une enzyme variante, éventuellement un variant d'une pullulanase de *Bacillus deramificans* ayant une séquence d'acides aminés de la SEQ ID NO: 6 et ayant des délétions, des additions, des insertions ou des substitutions par comparaison avec la séquence d'acides aminés de la SEQ ID NO: 6.

11. Procédé selon la revendication 10, la pullulanase variante présentant des propriétés modifiées par comparaison avec une pullulanase ayant une séquence d'acides aminés de la SEQ ID NO: 6.

12. Procédé selon la revendication 11, les propriétés modifiées comprenant une stabilité thermique améliorée, une activité dépendant du pH améliorée, une activité spécifique améliorée, une spécificité pour un substrat améliorée ou une quelconque combinaison correspondante, par comparaison avec une pullulanase ayant une séquence d'acides aminés de la SEQ ID NO: 6.

13. Procédé selon la revendication 1, la pullulanase étant exprimée et/ou isolée à partir d'une cellule hôte de *Bacillus,* et éventuellement à partir de la cellule hôte de *Bacillus licheniformis.*

14. Procédé selon la revendication 13, un gène de protéase Carlsberg et/ou un gène de protéase Glu C de la cellule hôte de *Bacillus licheniformis* ayant été modifiés pour éliminer une activité de protéase.

15. Procédé selon la revendication 1, le variant de PS4 et/ou la pullulanase étant purifiés.

16. Procédé selon la revendication 1, le variant de PS4 étant ajouté au produit de liquéfaction d'amidon en une plage de 0,05 à 1 kg/tonne de solides secs.

17. Procédé selon la revendication 1, la pullulanase, mesurée en kg/tonne de solides secs, étant ajoutée au produit de liquéfaction d'amidon à raison d'une quantité dans une plage de 5 à environ 50 fois le variant de PS4.

18. Procédé selon la revendication 1,
(a) le produit de liquéfaction d'amidon étant saccharifié à une température de 60 °C à 75 °C ; et/ou
(b) le produit de liquéfaction d'amidon étant saccharifié à un pH 3,9 à pH 5,5.

19. Procédé selon la revendication 1, le sirop de saccharide contenant au moins 20 % en plus de maltotétraose qu'un sirop de saccharide obtenu avec le variant de PS4 mais sans la pullulanase.

20. Procédé selon la revendication 1 comprenant en outre la mise en contact d'une isoamylase, d'une protéase, d'une cellulase, d'une hémicellulose, d'une lipase, d'une cutinase, ou d'une quelconque combinaison correspondante, avec le produit de liquéfaction d'amidon ou la maltodextrine.

21. Procédé selon la revendication 1, l'amidon provenant de maïs, de blé, d'orge, de seigle, de milo, de sagou, de manioc, de tapioca, de sorgho, de riz, de petit pois, de haricot, de banane ou de pommes de terre.
